# EUROPEAN PATENT SPECIFICATION

(11) **EP 2 207 885 B1**
(45) Date of publication and mention of the grant of the patent: **20.11.2013**
(21) Application number: 08843771.0
(22) Date of filing: 23.10.2008
(51) Int. Cl.: C07K 14/47, C07K 16/18, A01K 67/027, C12N 15/85

(54) **QUADRUPLE TRANSGENIC NON-HUMAN ANIMAL**
VIERFACH TRANSGENES NICHTMENSCHLICHES TIER
ANIMAL NON HUMAIN QUADRUPLEMENT TRANSGÉNIQUE

(30) Priority: 01.11.2007 EP 07119837
(43) Date of publication of application: 21.07.2010
(73) Proprietor: F. Hoffmann-La Roche AG, 4070 Basel (CH)
(72) Inventor: BOHRMANN, Bernd, CH-4125 Riehen (CH); IGLESIAS, Antonio, 79102 Freiburg (DE); LOETSCHER, Hansruedi, CH-4313 Moehlin (CH)
(74) Representative: Küng, Peter
(86) International application number: PCT/EP2008/008971
(87) International publication number: WO 2009/056257

(56) References cited:
- EP-A- 1 917 854
- WO-A-01/97607
- WO-A-02/05634
- WO-A-03/070760

## Description

Alzheimer's disease (AD) is a neurodegenerative disease which is characterized by memory loss and decline of cognitive functions. The prevalence of AD approximately doubles with every five years over the age of 65. Overall, approximately 5 to 10% of those over 65 are affected. The clinical diagnosis of AD remains an exclusion diagnosis. Only post-mortem microscopic examination of the brain offers a definitive diagnosis based on the presence of extracellular plaques containing fibrils of amyloid-beta (A-beta) peptide and intracellular tangles containing polymerized phosphorylated Tau protein. In most of the cases, AD occurs late in onset and sporadically. The early onset familial AD represents a minority of the cases, but they have been extremely important for the progression in the understanding of the disease mechanisms. Known mutations are the Swedish Mutation (SFAD) which affect codons 670/671 (K670+N and M671+L) and the London mutation which affects codon 717 of APP. All these mutations affect the proteolytic processing of APP yielding more amyloidogenic peptides.

APP can be processed by at least 3 secretases: alpha-, beta-, and gamma-secretases. Beta-secretase initiates A-beta peptide generation by cleaving APP after Methionine 671 (APP770 numbering) leading to a 12kd retained membrane carboxyterminal fragment. The 12kd fragment may then undergo gamma-secretase cleavage within the hydrophobic transmembrane domain to release the 40, 42, or 43 residue A-beta peptides (Seubert P, Vigo-Pelfrey C, Esch F, Lee M, Dovey H, Davis D, Sinha S, Schlossmacher M, Whaley J, Swindlehurst C. Isolation and quantification of soluble Alzheimer's beta-peptide from biological fluids. Nature. 1992. 359: 325-7).

Current treatments for AD provide only modest symptomatic relief. There is a high unmet medical need for disease modifying agents that slow the course of the disease and prevent or delay the disease in susceptible individuals. The development of such agents requires, among others, progress in the understanding of the molecular basis of the disease and in the development of animal models.

A number of transgenic lines overexpressing proteins relating with AD are known, for example double transgenic mice overexpressing APP London and beta secretase.

The present invention provides a transgenic mouse whose genome comprises
a) a first transgenic DNA sequence encoding a human APP Swedish or a human APP London protein, wherein said first transgenic DNA sequence is operably linked to a first promoter;
b) a second transgenic DNA sequence encoding a human Presenilin 2 protein comprising a N141I substitution, wherein said second transgenic DNA sequence is operably linked to a second promoter;
c) a third transgenic DNA sequence encoding a light chain of an antibody directed against the amyloid peptide, wherein said third transgenic DNA sequence is operably linked to a third promoter and;
d) a fourth transgenic DNA sequence encoding a heavy chain of said antibody, wherein said forth transgenic is operably linked to the third promoter or to a forth promoter.

The term "Alzheimer's disease (AD) pathology" refers to pathological changes in AD patients such as for example loss of neuronal synaptic density and synapse number, decreased cognitive ability, and memory loss. They also include diffuse and neuritic plaques in the brain, which are predominantly composed of amyloid beta peptide, neuronal and/or glial inclusions or insoluble deposits that stain positively with anti-Abeta antibodies.

In particular, the present invention provides a transgenic mouse whose genome comprises
a) a first transgenic DNA sequence encoding a human APP Swedish or a human APP London protein, wherein said first transgenic DNA sequence is operably linked to a first promoter;
b) a second transgenic DNA sequence encoding a human Presenilin 2 protein comprising a N141I substitution, wherein said second transgenic DNA sequence is operably linked to a second promoter;
c) a third transgenic DNA sequence encoding a light chain of an antibody directed against the amyloid peptide, wherein said third transgenic DNA sequence is operably linked to a third promoter and;
d) a fourth transgenic DNA sequence encoding a heavy chain of said antibody, wherein said forth transgenic is operably linked to the third promoter or to a forth promoter.

### APP

APP means amyloid precursor protein. A number of cDNA forms of APP have been identified, encoding among others the three most abundant isoforms APP695, APP751, and APP770. These forms arise from a single precursor RNA by alternate splicing. The gene spans more than 175 kb with 18 exons (Yoshikai S, Sasaki H, Doh-ura K, Furuya H, Sakaki Y. Genomic organization of the human amyloid beta-protein precursor gene. Gene. 1990. 87(2):257-63). APP contains an extracellular domain, a transmembrane region and a cytoplasmic domain. A-beta consists of up to 28 amino acids just outside the hydrophobic transmembrane domain and up to 15 residues of this transmembrane domain. Thus, A-beta is a cleavage product derived from APP which is normally found in brain and other tissues such as heart, kidney and spleen. However, A-beta deposits are usually found in abundance only in the brain. The larger alternate forms of APP (APP751, APP770) consist of APP695 plus one or two additional domains. APP751 consists of all 695 amino acids of APP695 plus an additional 56 amino acids which has homology to the Kunitz family of serine protease inhibitors (KPI) (Tanzi RE, McClatchey AI, Lamperti ED, Villa-Komaroff L, Gusella JF, Neve RL. Protease inhibitors domain encoded by an amyloid protein precursor mRNA associated with Alzheimer's disease. Nature. 1988. 331:528-30*;* Weidemann A, Konig G, Burke D, Fischer P, Salbaum JM, Masters CL, Beyreuther K. Identification, biogenesis, and localization of precursors of Alzheimer's disease A4 amyloid protein. Cell. 1989 Apr 7; 57(1):115-26*.* Kitaguchi N, Takahashi Y, Tokushima Y, Shiojiri S, Ito H. Novel precursor of Alzheimer's disease amyloid protein shows protease inhibitory activity. Nature. 1988. Feb 11;331(6156):530-2*.;* Tanzi RE, St George-Hyslop PH, Haines JL, Polinsky RJ, Nee L, Foncin JF, Neve RL, McClatchey AI, Conneally PM, Gusella JF. The genetic defect in familial Alzheimer's disease is not tightly linked to the amyloid beta-protein gene. Nature. 1987. 329 (6135):156-7*).* APP770 contains all 751 amino acids of APP751 and an additional 19 amino acid domain homologous to the neuron cell surface antigen OX-2 (Weidemann et al. 1989; Kitaguchi et al. 1988). Unless otherwise noted, the amino acid positions referred to herein are the positions as they appear in APP770. The amino acid number of equivalent positions in APP695 and APP751 differ in some cases due to the absence of the OX-2 and KPI domains. By convention, the amino acid positions of all forms of APP are referenced by the equivalent positions in the APP770 form. Unless otherwise noted, this convention is followed herein. Unless otherwise noted, all forms of APP and fragments of APP, including all forms of A-beta, referred to herein are based on the human APP amino acid sequence. APP is post-translationally modified by the removal of the leader sequence and by the addition of sulfate and sugar groups. The term "APP" includes also mutant APP, such for example as APPswedish and APP London.

A human APP protein comprising a mutation associated with Alzheimer's disease (AD) or AD-type pathology is preferably APPswedish and APPlondon.

The term "APP Swedish" as used herein refers an APP isoform as defined above comprising a K670N/M671L substitution. Preferably the human APP Swedish is encoded by SEQ. ID NO: 1.

The term "APP London" as used herein refers to an APP isoform as defined above containing one or more of the either natural or artificial mutations which affect the production of amyloid peptides towards a specific increased production of A-beta 42 peptide. Preferred are natural APP mutations around the gamma-secretase cleavage site with pathological relevance including
- T714I (Kumar-Singh S et al. Hum Mol Genet. 2000 Nov 1; 9(18):2589-98.)
- V715M (Ancolio K, et al., Proc Natl Acad Sci U S A. 1999 Mar 30;96(7):4119-24.)
- I716V (Eckman CB, et al., Hum Mol Genet. 1997 Nov; 6(12):2087-9.)
- V717F (Murrell J, et al, Science. 1991 Oct 4; 254(5028):97-9.)
- V717G (Chartier-Harlin MC, et al., Nature. 1991 Oct 31; 353(6347):844-6.)
- V717I (Goate A, et al., Nature. 1991 Feb 21;349(6311):704-6)
- V717L (Murrell JR, et al., Arch Neurol. 2000 Jun;57(6):885-7.)
- L723P (Kwok JB, et al., Ann Neurol. 2000 Feb;47(2):249-53.)

Preferred are also artificial mutations (replacement of all residues in the transmembrane region outside of the A-beta domain) examples of which and their consequence on the gamma-secretase cleavage have been described by Lichtenthaler and co-workers (Lichtenthaler SF, et al., Proc Natl Acad Sci U S A. 1999 Mar 16;96(6):3053-8).

More preferably, human APP London is human APP London containing the natural V717F mutation. Most preferably the human APP London is encoded by SEQ ID NO: 3.

Preferably, the first transgenic DNA sequence encodes human APPswedish.

### Presenilin 2 (PS2)

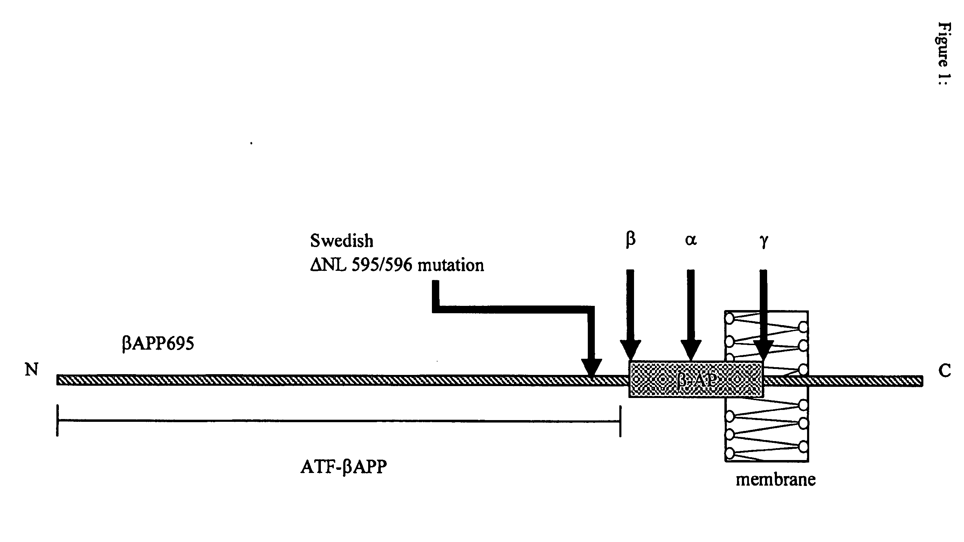

Presenilin 2 (PS) is a protein of the gamma-secretase complex. The gamma-secretase is involved in the processing of the APP (see Figure 1).

The term "DNA sequence encoding Presenilin 2" or "PS2 gene" as used herein refers to the mammalian gene first disclosed and described in U.S. Application Ser. No. 08/496,841, filed on June 28, 1995, and later described in Rogaev et al. (Nature. 1995; 376(6543):775-8) and Levy Lahad et al. (Ann Neurol. 1995; 38(4):678-80), including any allelic variants and heterospecific mammalian homologues. One human presenilin-2 (hPS2) cDNA sequence is disclosed herein as SEQ ID NO: 9.

The term "presenilin-2 gene" or "PS2 gene" primarily relates to a coding sequence, but can also include some or all of the flanking regulatory regions and/or introns. The term PS2 gene specifically includes artificial or recombinant genes created from cDNA or genomic DNA, including recombinant genes based upon splice variants. The presenilin-2 gene has also been referred to as the E5-1 gene.

A human Presenilin 2 protein comprising a mutation associated with Alzheimer's disease (AD) or AD-type pathology is preferably a human PS2 with N141I, T122P, M239V or M239I substitution (Shen et al., PNAS, 2007, 104(2):403-409). More preferable, the human Presenilin 2 protein comprising a mutation associated with Alzheimer's disease (AD) or AD-type pathology is a human PS2 with N141I substitution.

Therefore, the second transgenic DNA sequence preferably encodes a human PS2 with a N141I substitution. Most preferred is the DNA sequence encoding SEQ. ID NO: 19

### Antibody

An antibody is a "Y"-shaped molecule and consists of two heavy chains and two light chains. There are different types and subtypes of both, the heavy chains and the light chains. Each heavy chain and each light chain has a constant region and a variable region, whereby the size of constant regions is bigger for the heavy chain.

The term "constant region" or "C region" refers to a region of an antibody molecule that is nearly identical with the corresponding regions of antibodies of different specificities produced by organisms of the same species. The constant part is invariable within the same antibody class (isotype) and is responsible for the effector functions of a particular Immunoglobulin subclass. An Fc fragment is a fragment of an antibody generated by the cleavage of an antibody with the enzyme papain and comprising the most part of the constant regions.

The term "variable region" as used herein refers to a region of an antibody molecule which binds to specific antigens. It is composed of the combination of the antigen-binding sites of the heavy and of the light chain. It differs between immunoglobulins of different B cells, but is the same for all immunoglobulins produced by the same B cell. The variable region is generated somatically via a gene recombination process taking place during maturation of B-cells. It is this process of rearrangement that creates the enormous diversity needed to bind any given antigen, and that thus enables the immune system to recognize and neutralize the innumerable antigenic burdens posed by foreign and pathogenic structures. As a consequence, the antibody pool is composed of immunoglobulins bearing a large repertoire of different V regions, while sharing the same Fc portions.

The term "fragment binding antigen" or "Fab fragment" is an antibody fragment that includes the variable, antigen-binding region, and which is generated by the cleavage of an antibody with the enzyme papain.

The term "idiotypic region" as used herein refers to the part of the variable region of an antibody that is unique for each antibody type.

The third transgenic nucleotide sequence encodes the light chain of antibody directed against the beta-amyloid peptide. Preferably, said antibody is a human, humanized or chimeric antibody. More preferably, said antibody is an immunoglobulin gamma (IgG). Most preferably, said antibody is the therapeutic antibody Mab11 (WO 03/070760).

The forth transgenic nucleotide sequence encodes the heavy chain of an antibody directed against the beta-amyloid peptide. Preferably, said antibody is a human, humanized or chimeric antibody. More preferably, said antibody is an immunoglobulin gamma (IgG). Most preferably, said antibody is the therapeutic antibody Mab11 (WO 03/070760).

The term "beta-amyloid peptide" or "A-beta" refers to a peptide produced by cleavage of APP by beta-secretase and gamma-secretase. The peptide has 39-43 amino acids. It is the main constituent of amyloid plaques in the brains of Alzheimer's disease patients. Preferably, the beta-amyloid peptide is a human beta-amyloid peptide.

Preferably, the forth transgenic nucleotide sequence encode for the heavy chain of a secreted antibody. Such a preferred DNA sequence lack sequences encoding a transmembrane domain.

The third and forth transgenic DNA sequences may be operably linked with one promoter (i.e. with the third promoter) or they may be operably linked each with a separate promoter (i.e. the third transgenic DNA sequence is operably linked with the third promoter and the forth DNA sequence is operably linked with the forth promoter).

### Promoters

The term "operably linked" as used herein means that a DNA sequence and a regulatory sequence are connected in a way as to permit gene expression when the appropriate transcriptional activator proteins are bound to the regulatory sequence.

The term "regulatory sequence" as used herein refers to is a nucleotide sequence which is a binding site for proteins controlling the protein expression. A regulatory sequence may for example a promoter or an enhancer.

The term "promoter" or as used herein refers region of gene that binds RNA polymerase and transcription factors to initiate transcription.

The term "enhancer" as used herein refers to a nucleotide sequence that increases the rate of genetic transcription by increasing the activity of the nearest promoter on the same DNA molecule

The first promoter may be any promoter which expresses the operably linked DNA in the neurons. The first promoter may be an ubiquitary promoter. Preferably, the first promoter is a neuronal promoter. More preferably, the first promoter is a brain- specific promoter.

The term "tissue-specific promoter" refers to a promoter, which controls and directs expression of a gene in a tissue-specific manner, e.g., in brain tissue, in muscle tissue, in liver tissue, in kidney tissue, etc. A brain-specific promoter is a promoter which controls ad directs expression of a gene in brain tissue. Most preferably, first promoter is a promoter of a prion gene.

The first promoter may also be a controllable promoter. A controllable promoter may be any promoter, which controls the expression of a transgene in a regulatable and/or inducible fashion, e.g., by addition of specific inducer or repressor substances. Several inducible bacterial promoter are known in the art (Schultze N, Burki Y, Lang Y, Certa U, Bluethmann H; Nat Biotechnol 1996; 14(4): 499-503; van der Neut R; Targeted gene disruption: applications in neurobiology; J Neurosci Methods 1997; 71(1): 19-27; Liu HS, Lee CH, Lee CF, Su U, Chang TY; Lac/Tet dual-inducible system functions in mammalian cell lines. Biotechniques. 1998; 24(4): 624-8, 630-2).

The **second** promoter may be any promoter which expresses the operably linked DNA in the neurons. The first promoter may be an ubiquitary promoter. Preferably, the second promoter is a neuronal promoter. More preferably, the second promoter is a brain-specific promoter.

The term "tissue-specific promoter" refers to a promoter, which controls and directs expression of a gene in a tissue-specific manner, e.g., in brain tissue, in muscle tissue, in liver tissue, in kidney tissue, etc. A brain-specific promoter is a promoter which controls ad directs expression of a gene in brain tissue. Most preferably, the second promoter is a Thy1.2 glycoprotein promoter.

The second promoter may also be a controllable promoter. A controllable promoter may be any promoter, which controls the expression of a transgene in a regulatable and/or inducible fashion, e.g., by addition of specific inducer or repressor substances. Several inducible bacterial promoter are known in the art (Schultze N, Burki Y, Lang Y, Certa U, Bluethmann H; Nat Biotechnol 1996; 14(4): 499-503; van der Neut R; Targeted gene disruption: applications in neurobiology; J Neurosci Methods 1997; 71(1): 19-27; Liu HS, Lee CH, Lee CF, Su IJ, Chang TY; Lac/Tet dual-inducible system functions in mammalian cell lines. Biotechniques. 1998; 24(4): 624-8, 630-2).

The **third** promoter may an ubiquitary promoter or a lymphoid-tissue specific promoter.

Preferably, the third promoter is a promoter of the MHC class I gene. Also preferably, the third transgenic DNA sequence is operably linked with an enhancer. More preferably, the enhancer is an IgH enhancer.

The third promoter may also be a controllable promoter. A controllable promoter may be any promoter, which controls the expression of a transgene in a regulatable and/or inducible fashion, e.g., by addition of specific inducer or repressor substances. Several inducible bacterial promoter are known in the art (Schultze N, Burki Y, Lang Y, Certa U, Bluethmann H; Nat Biotechnol 1996; 14(4): 499-503; van der Neut R; Targeted gene disruption: applications in neurobiology; J Neurosci Methods 1997; 71(1): 19-27; Liu HS, Lee CH, Lee CF, Su IJ, Chang TY; Lac/Tet dual-inducible system functions in mammalian cell lines. Biotechniques. 1998; 24(4): 624-8, 630-2).

The **forth** promoter may an ubiquitary promoter or a lymphoid-tissue specific promoter. Preferably, the forth promoter is a promoter of the MHC class I gene. Also preferably, the fourth transgenic DNA sequences is operably coupled with an enhancer. More preferably, the enhancer is an IgH enhancer.

The fourth promoter may also be a controllable promoter. A controllable promoter may be any promoter, which controls the expression of a transgene in a regulatable and/or inducible fashion, e.g., by addition of specific inducer or repressor substances. Several inducible bacterial promoter are known in the art (Schultze N, Burki Y, Lang Y, Certa U, Bluethmann H; Nat Biotechnol 1996; 14(4): 499-503; van der Neut R; Targeted gene disruption: applications in neurobiology; J Neurosci Methods 1997; 71(1): 19-27; Liu HS, Lee CH, Lee CF, Su U, Chang TY; Lac/Tet dual-inducible system functions in mammalian cell lines. Biotechniques. 1998; 24(4): 624-8, 630-2).

As the transgenic mouse produces also its own antibodies, the human heavy and light chains may be mixed up with the endogenous heavy and light chains to form a mixed endogenous/transgenic antibody. In case of a mouse, mixed mouse/human antibodies may be formed. If the human heavy and light chains are produce outside the lymphoid tissue, no endogenous heavy and light chains will be present and therefore pure transgenic antibodies will be formed. Therefore, the promoter(s) operably linked to the third and fourth transgenic DNA sequence is preferably a promoter expressed in both, lymphoid and non-lymphoid tissue.

### Transgenic animal / Integration of the transgenes

The transgenic DNA sequences are integrated in all or a portion of the animals cells, especially in the germ cells. The integration into the genome may be transient or stable. Preferably, the integration is stable.

The transgenic mouse may be heterozygous or homozygous for the first, second, third and forth transgenic DNA sequences. The transgenic mouse may be heterozygous for some of the transgenic DNA sequences, for example for the first and second transgenic DNA sequences, and homozygous for the other, for example for the third and fourth DNA sequences. Preferably, the transgenic mouse is homozygous for at least one of the transgenic DNA sequences.

### Production of transgenic animals

Methods for producing a transgenic non-human animal are well known in the art. Suitable methods are described i.e. in Hogan B., Beddington R., Costantini F. & Lacy E. Manipulating the mouse embryo. A laboratory manual. 2nd Edition (1994). Cold Spring Harbor Laboratory Press.

The transgenic proteins may be ubiquitary expressed. Preferably, the proteins encoded by the first and second transgenic DNA sequence are expressed in the neurons. More preferably, the proteins encoded by the first and second transgenic DNA sequence are expressed in brain-tissue. Preferably, the proteins encoded by the third and forth transgenic DNA sequences are expressed in lymphoid tissue. More preferably, the proteins encoded by the third and fourth transgenic DNA sequences are expressed in both, lymphoid and non-lymphoid tissue.

The transgenic non-human mice described above may be analyzed genetically, molecularly and behaviorally.

### Use

The quadruple transgenic mouse as described above can be used as a model for treatment of the Alzheimer's disease or as model for preventing AD. It allows to investigate the effect of an human anti-Aβ antibody on the onset and process of Aβ amyloidosis in a murine AD model. Since the transgenic animal is immunotolerant for the transgenic human mAb11 antibody, the effect of chronic treatment with a therapeutic antibody such as for example Mab 11 can be determined. Also, the effect on soluble Aβ levels and the process and kinetics of vascular amyloidosis can be followed.

Furthermore, the quadruple transgenic mouse as described above is an interesting tool for determining the effectiveness and effect of a preventive treatment of Alzheimer's disease with a human antibody.

Having now generally described this invention, the same will become better understood by reference to the specific examples, which are included herein for purpose of illustration only and are not intended to be limiting unless otherwise specified, in connection with the following figures.

### Figures

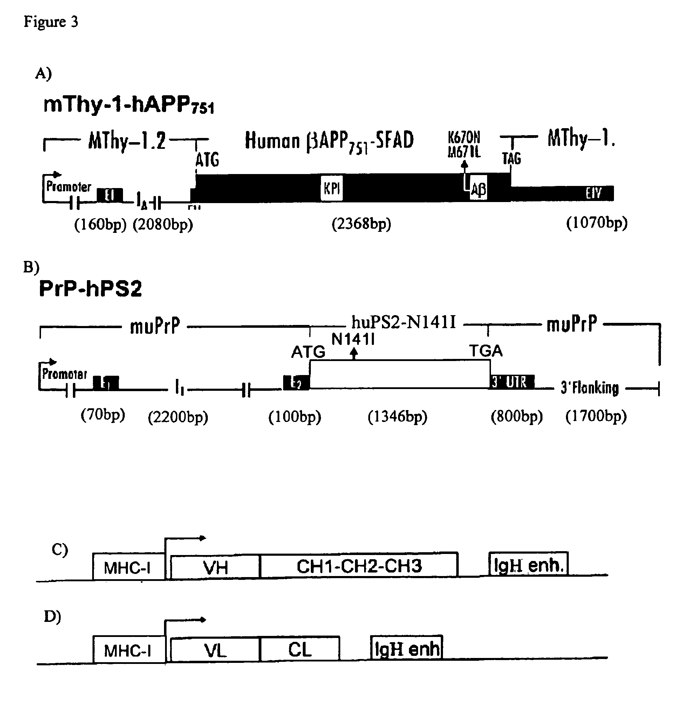

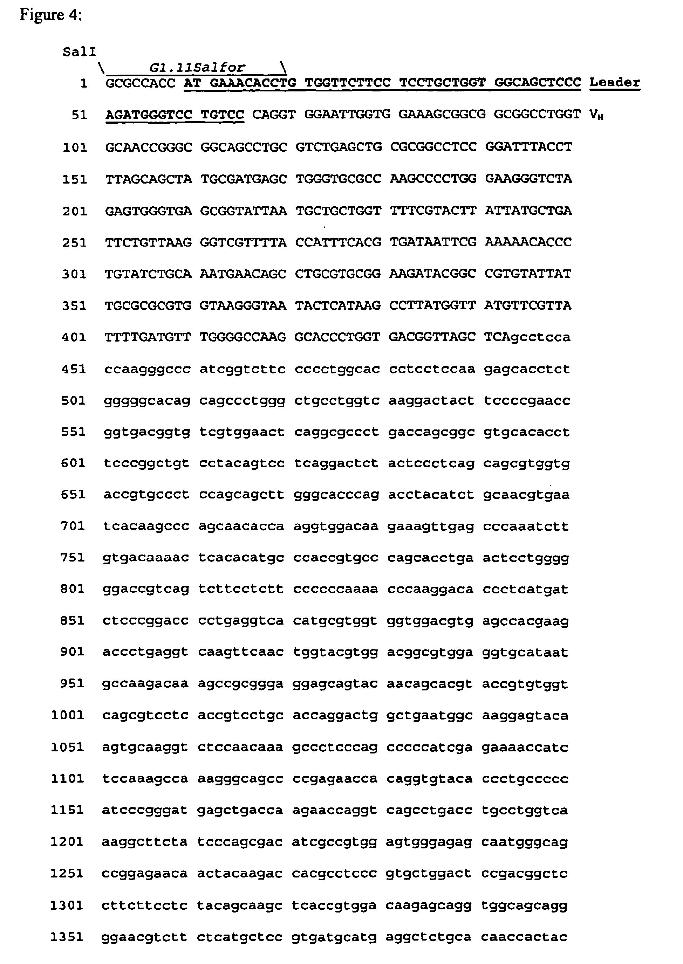

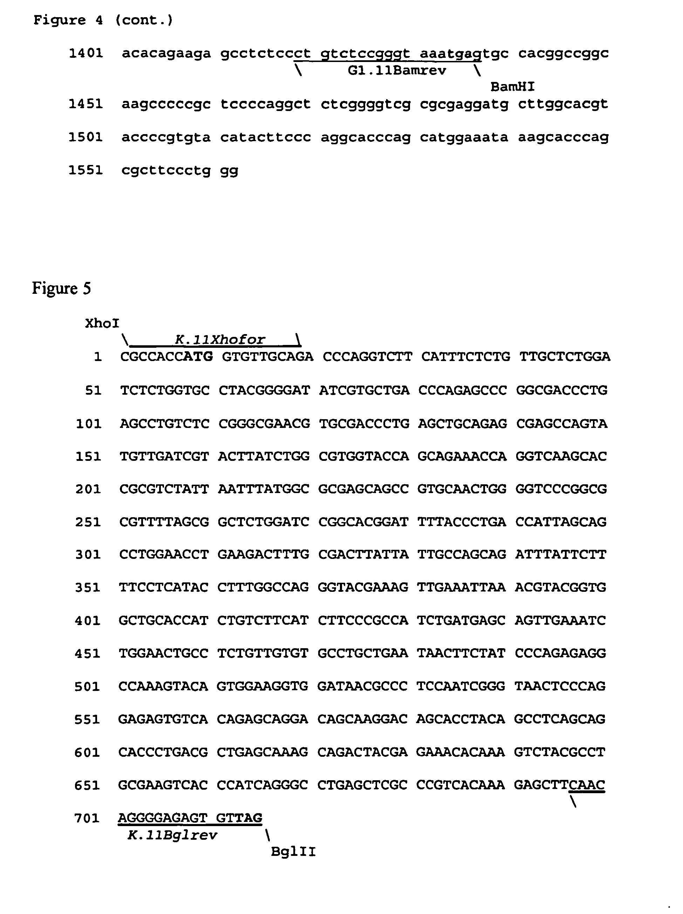

Figure 1 shows a schematic representation of the APP processing. A) Amyloid precursor protein. The cleavage site of the three secretases are indicated (α: α secretase, β: β secretase, γ: γ secretase).

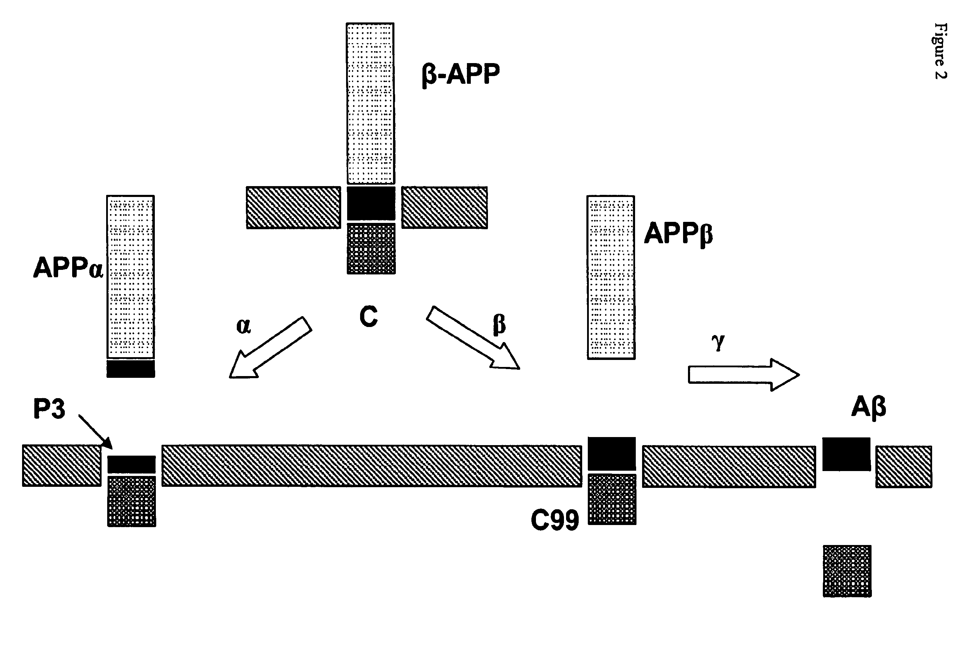

Figure 2 shows a schematic representation of the amyloid precursor protein (APP) processing. APP can undergo proteolytic processing via 2 pathways. Cleavage by α-secretase occurs within the A-beta domain and generates the large soluble N-terminal APPα and a non-amyloidogenic C-terminal fragment. Further proteolysis of this fragment by g-secretase generates the non-amyloidogenic peptide p3. Alternatively, cleavage of APP by β-secretase occurs at the beginning of the Aβ domain and generates a shorter soluble N-terminus, APPβ, as well as an amyloidogenic C-terminal fragment (C99). Further cleavage of this C-terminal fragment by γ-secretase generates Aβ. Cleavage by γ-secretase or multiple γ-secretases can result in C-terminal heterogeneity of Aβ to generate Aβ40 and Aβ42.
   ▨ Cell membrane; 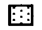 APPβ, ■ Aβ, ▦ CTF
Figure 3 shows a schematic representation of the genetic constructs.
   A) Construct comprising DNA encoding human betaAPP751 with K60N, M671L substitution (human βAPP751-SFAD) embedded in the mouse Thy 1.2 glycoprotein gene (MThy1.2). KPI: Kunitz protease inhibitor domain; Aβ: beta amyloid peptide; EI: Exons I; EII: Exon II; EIV: Exon IV; ATG: start codon; TAG: stop codon.
   B) Construct comprising the DNA encoding a mutant human presenilin 2 with N141 substitution (hPS2-N141I) embedded in the mouse prion (pPrPHG). E1: Exon 1, E2: Exon 2, UTR: untranslational region; ATG: start codon; TAG: stop codon.
   C) Construct comprising DNA encoding the heavy chain of the human antibody IgG1.1 (Mab11) directed against the beta-amyloid peptide. MHC-I: H-2k promoter region, VH: Variable regions of the heavy chain of the antibody, CH1-CH2-CH3: Constant regions of the heavy chain of the antibody, IgH enhancer.: transcriptional enhancer element of the murine IgH gene
   D) construct comprising DNA encoding the light chain of the human antibody IgG1.1 (Mab11) directed against the beta-amyloid peptide. MHC-I: H-2k promoter region, VL: Variable regions of the light chain of the antibody, CL: Constant regions of the light chain of the antibody, IgH enhancer: transcriptional enhancer element of the murine IgH gene
Figure 4 shows the entire coding sequence (SEQ. ID NO: 10) of the human Ig γ1 gene specific for human Aβ as cloned into the expression vector pHSE3' for transgenic expression. The position and the name of the primers used in PCR amplification are displayed above or below the corresponding sequence. The Stop codon is shown in bold. Leader sequence is shown bold and underlined.
Figure 5 shows the entire coding sequence (SEQ. ID NO: 11) of the human Ig κ gene specific for human Aβ as cloned into the expression vector pHSE3' for transgenic expression. The position and the name of the primers used in PCR amplification are displayed above or below the corresponding sequence. The start and stop codon are shown in bold.

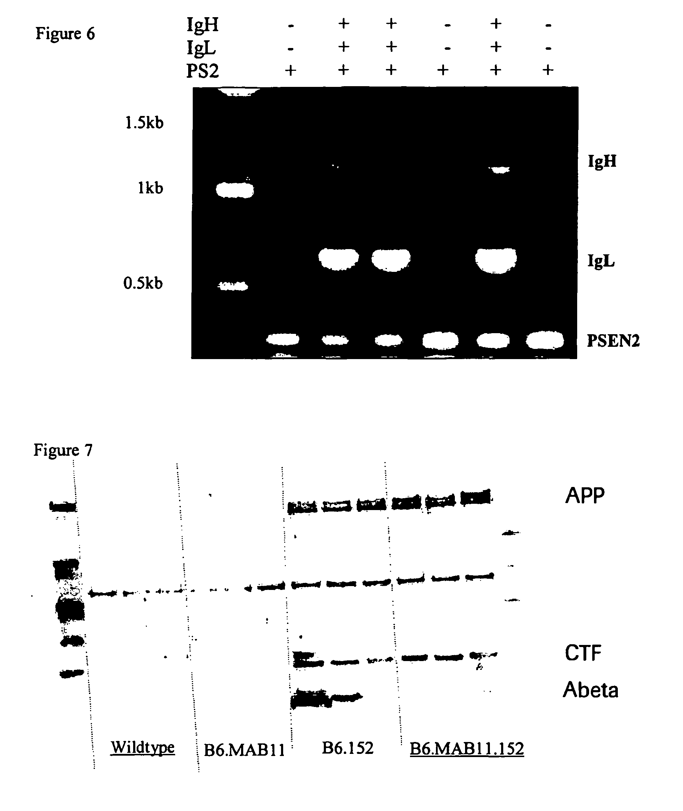

Figure 6 shows a picture of the PCR electrophoresis gel to detect the quadruple transgenic in progeny of B6.Mab11xB6.PS2APP mice. Lane 1: marker, lane 2 to 7: progeny mice of B6.Mab11xB6.PS2APP mice.IgH: IgH specific PCR fragment; IgL: IgL specific PCR fragment. PSEN2: PS(N41I) specific PCR fragment.
Figure 7 shows a Western Blot of brain homogenate from B6.MAB11, B6.152 and B6.MAB11.152 mice probed for APP.
   B6.MAB11: mouse transgenic for the heavy and light chain of MAB 11. B6.152: mouse transgenic for PS2 (N141I)/APPswedish. B6.MAB 11.152: mouse transgenic for the heavy and light chain of MAB 11 and for PS2 (N141I)/APPswedish. APP: Amyloid precursor protein, CTF: C-terminal fragment, Abeta: Beta amyloid peptide.

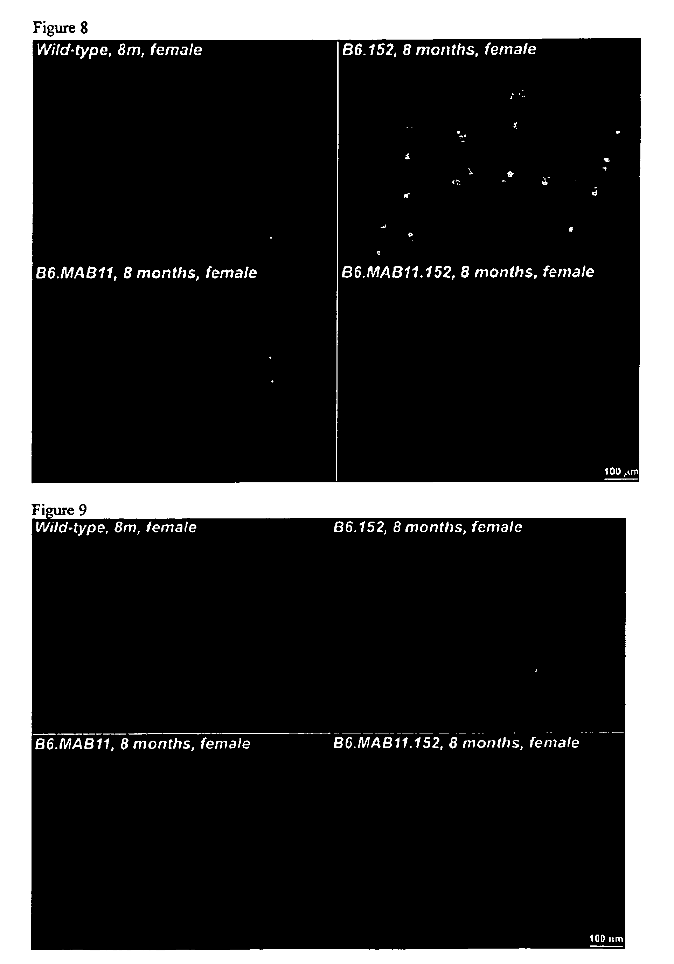

Figure 8 shows fluorescence microscopy images of tissue section of the frontal cortex of wildtype, Mab11 Transgenic mouse (B6.MAB11), PS2(N141I)/APPsw double transgenic mouse (B6.152) and the quadruple mouse transgenic for the heavy and light chain of MAB 11 and for PS2 (N141I)/APPswedish (B6.MAB11.152). The mice were 8 month old and the sections were stained with a murine anti-Ab monoclonal antibody (BAP2/Alexa 555 conjugate). The transgenic expression of MAB 11 in the quadruple transgenic mouse completely prevents plaque formation.
Figure 9 shows fluorescent microscopy images of tissue section of the frontal cortex of wildtype, Mab11 transgenic mouse (B6.MAB11), PS2(N141I)/APPsw double transgenic mouse (B6.152) and the quadruple mouse transgenic for the heavy and light chain of MAB 11 and for PS2 (N141I)/APPswedish (B6.MAB11.152). The mice were 8 month old and the sections were stained with thioflavin-S. The expression of MAB11 in the quadruple transgenic mouse completely prevents plaque formation.

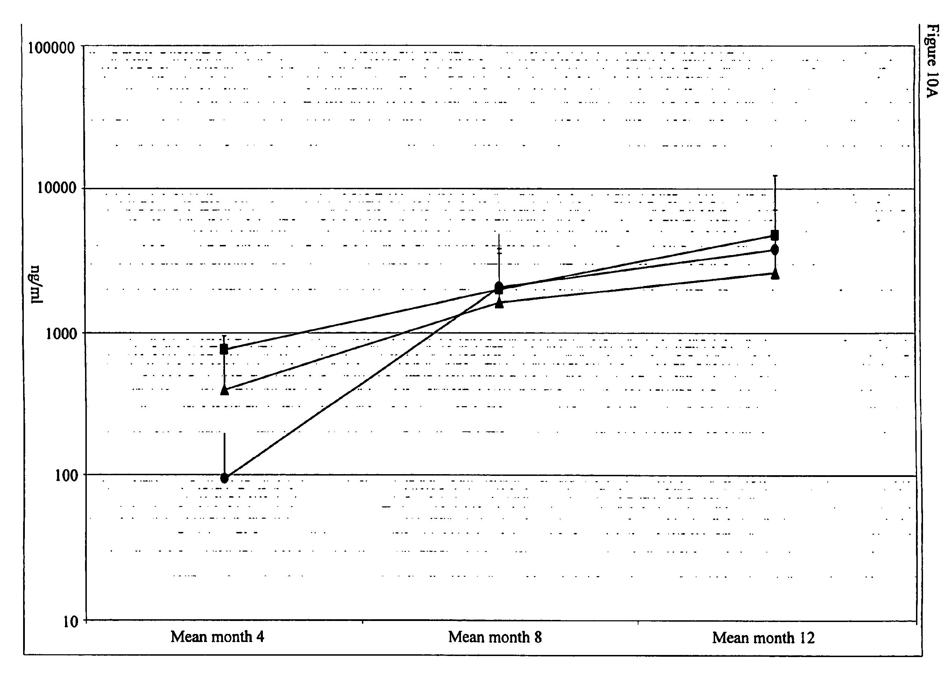

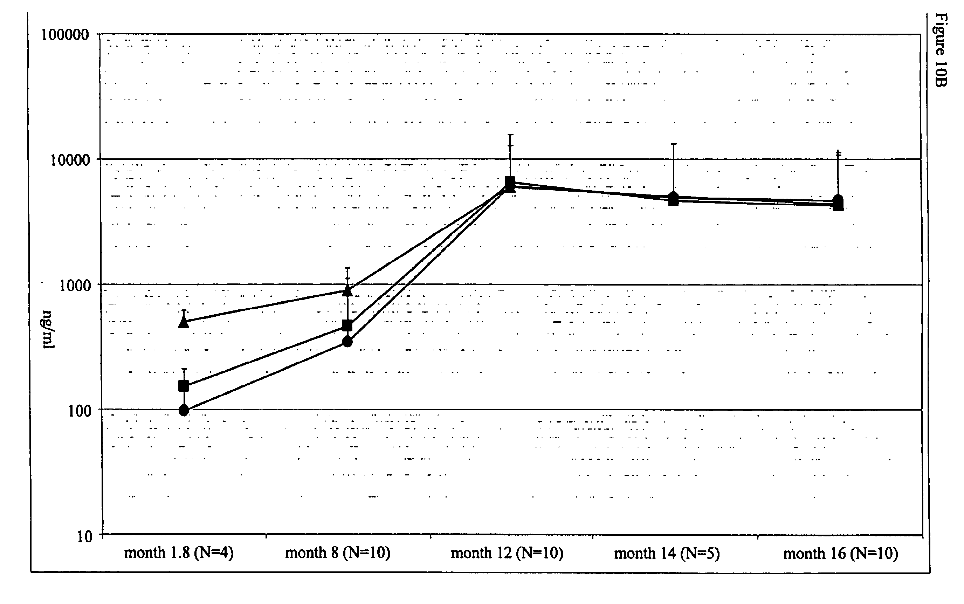

Figure 10 shows a graphical representation of measured levels of human IgG, i.e.MAB11 in plasma withdrawn from the Mab11 transgenic mouse (A) and the quadruple transgenic mouse (B6.MAB11.152) (B). Different independent methods were compared, (i) human IgG captured by Aβ antigen and detected by an Fc specific detection antibody -●-, (ii) human IgG captured by anti-human IgG (H+L) and detected by an (H+L) specific detection antibody -▲- and (iii) human IgG captured by anti-human IgG (H+L) and detected by an Fc specific detection antibody -■-.

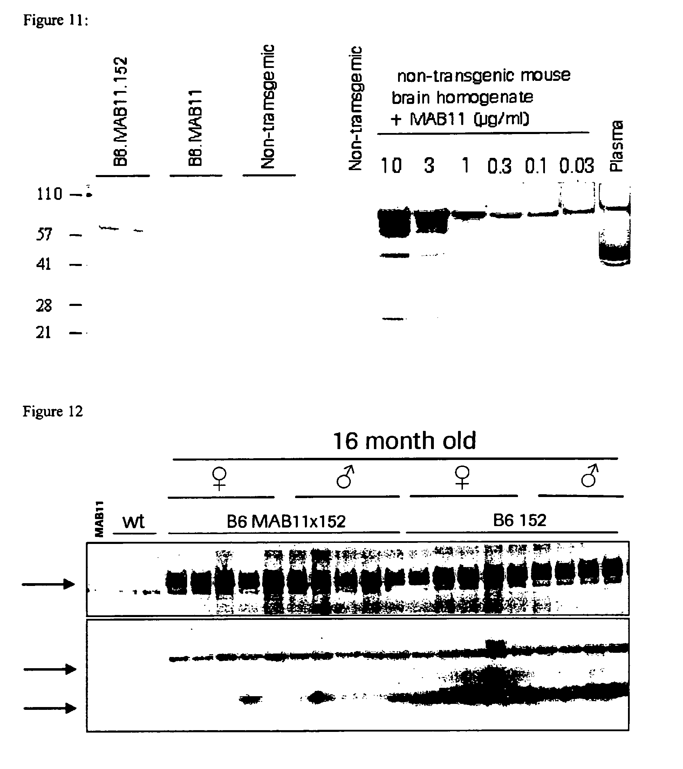

Figure 11 shows a Western Blot of brain homogenate from quadruple transgenic mouse B6.MAB11.152, the Mab11 transgenic mouse B6.MAB11 on a non-transgenic background (C57B1/6) probed with for human IgG Mab11. For determining the measurable minimal amount of Mab11, the antibody was added at different concentrations (10, 3, 1, 0.3, 0.1 and 0.03 µg/ml) to the brain homogenate of the non-transgenic mouse B6 (C57B1/6). Plasma withdrawn from the non-transgenic mouse is the control for Mab11 in the periphery.
Figure 12 shows a western blot of brain homogenates from 16 month old mice probed for Aβ. The brains were taken from non-transgenic wildtype C57B1/6 mice, of quadruple transgenic mice B6.MAB11.152 and PS2(N141I)/APPsw double transgenic mouse B6.152. In the first lane MAB 11 was added to sample buffer without brain homogenate as negative control.

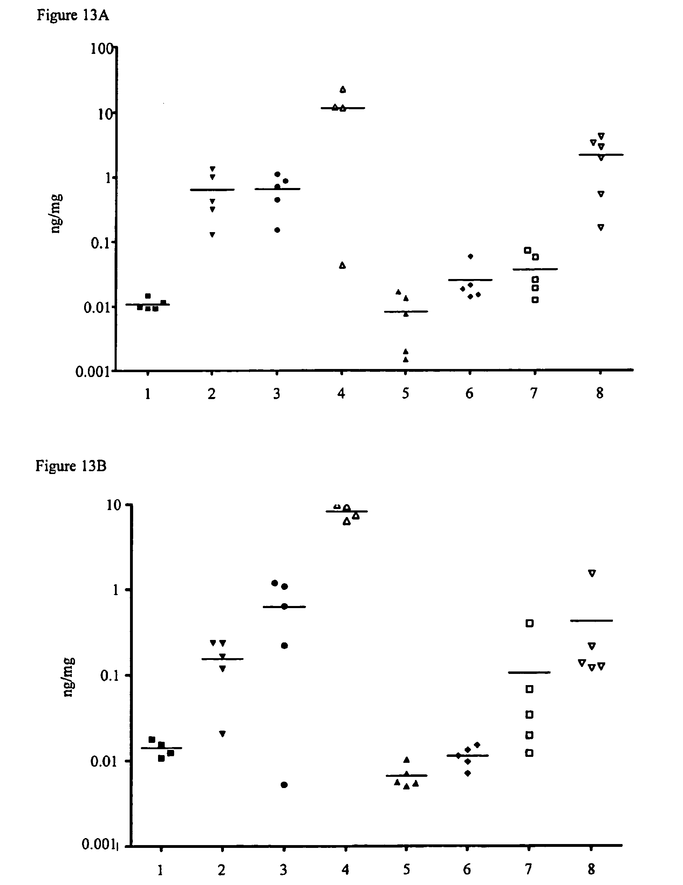

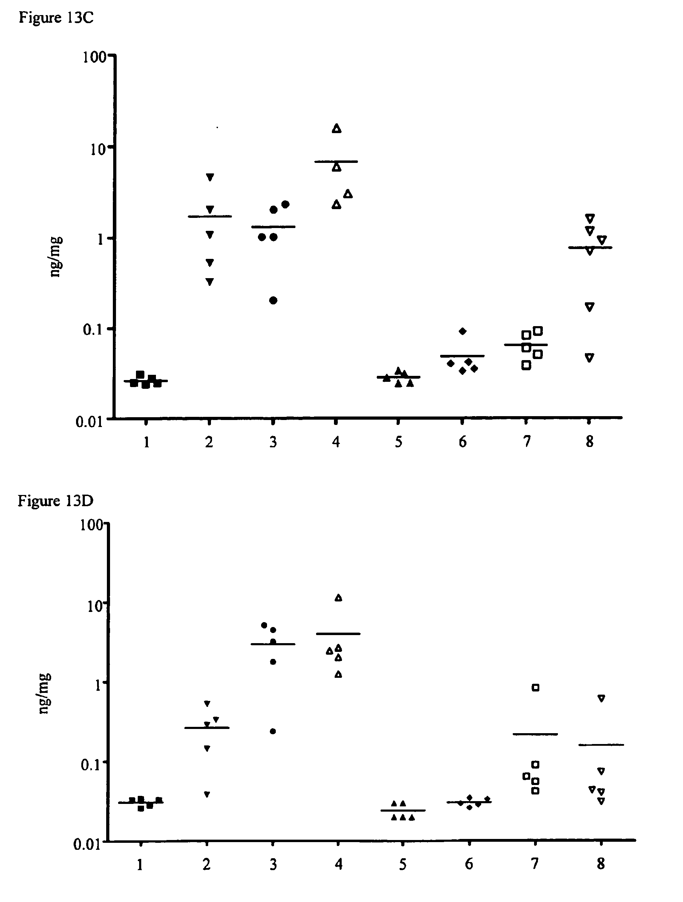

Figure 13 shows a graphical representation of measured unsoluble Abeta (Abeta40 and 42) after Aβ dissociation in the brain homogenate from B6.152 and B6.MAB 11.152 mice (in ng/mg brain).
   1-4: PS2(N141I)/APPsw double transgenic mouse (B6.152): 1 = 4 months old; 2 = 8 months old, 3 = 12 months old, 4 = 16 months old;
   5 - 8: quadruple transgenic mouse (B6.MAB11.152): 5 = 4 months old; 6 = 8 months old, 7 = 12 months old, 8 = 16 months old.
      A): Abeta 42 measured in females mice
      B) Abeta 42 measured in male mice
      C) Abeta 40 measured in female mice,
      D) Abeta 40 measured in male mice.

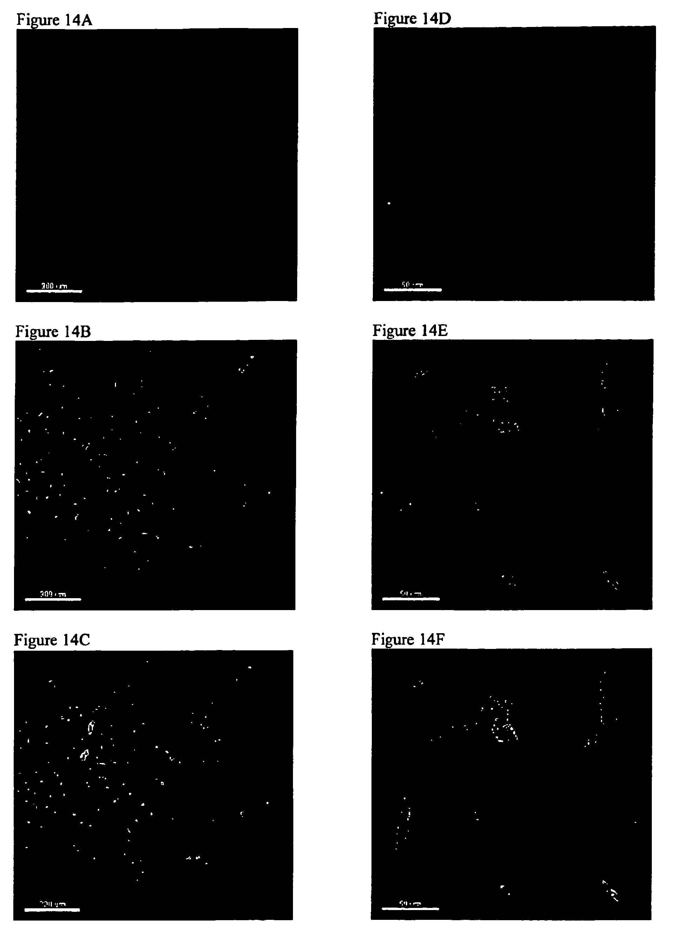

Figure 14 shows confocal scanning microscopy images visualized as maximal intensity projection of cortex sections taken from B6.MAB 11.152 double-immunostained for vascular amyloid deposits and endothelial cells. Figures 14A and D show BAP2 staining of amyloid deposition, staining at low and high magnification, respectively. For comparison, Figures 14B and E show the distribution of capillaries by CD31 (endothelial cell marker) staining at low and high magnification, respectively. Figure 14C and F show the merged channels of both stainings.

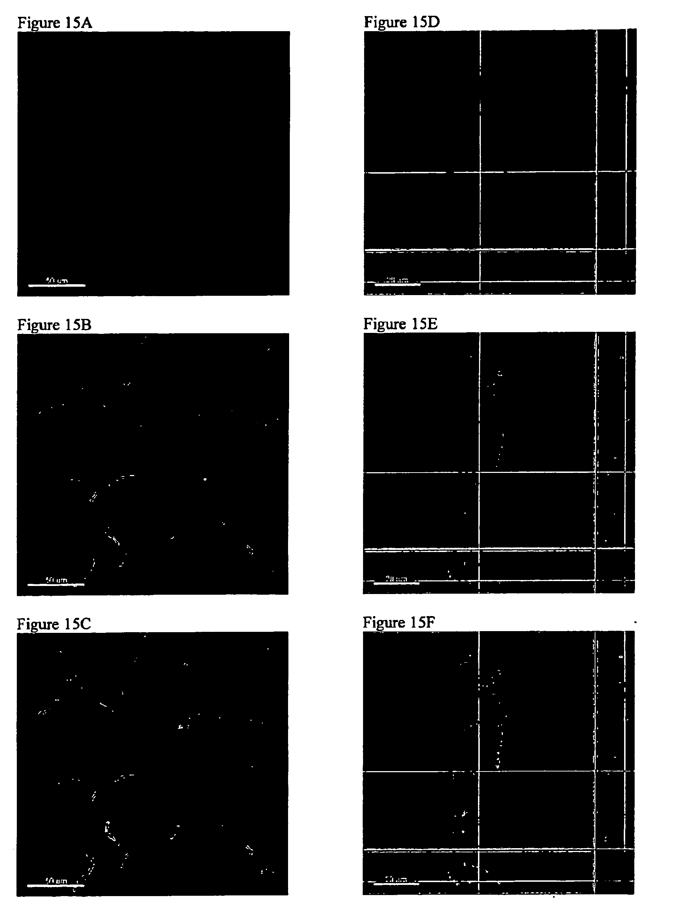

Figure 15 shows confocal scanning microscopy images visualized as maximal intensity projection taken from the double transgenic B6.152 mouse (A to C) and single confocal plane images of a cortical capillary from a quadruple transgenic B6.MAB 11.152 (D to F) at high resolution. Figures 15A and D show BAP2 staining of amyloid deposits. Figures 14B and E show CD31 (endothelial cell marker) staining. Figure 14C and F show the merged channels of both stainings.

### Examples:

Commercially available reagents referred to in the examples were used according to manufacturer's instructions unless otherwise indicated.

### Example 1: Transgenic constructs (Fig 1)

*APPSwe construct:* The cDNA encoding human βAPP751 was donated by Prof. D.Goldgaber. The Swedish familial AD double mutation (SFAD, K670N, M671L substitution) was introduced by site-directed mutagenesis. The Thy1.2-huβAPP751SFAD transgene was generated by insertion of the approximately 2.4kbp huβAPP751 SFAD cDNA fragment (SEQ. ID NO: 1: nucleotides 32-2369 of AC: X06989) into the XhoI site of an expression vector containing mouse Thy-1.2 glycoprotein gene and promoter (SEQ. ID NO:2, AC:M 12379). This expression vector has a 6.8kbp NotI fragment comprising mouse Thy1.2 gene (Andra K, et al., (1996) Expression of APP in transgenic mice: a comparison of neuron-specific promoters. Neurobiol Aging 17:183-190; Vidal M, et al., (1990) Tissue-specific control elements of the Thy-1 gene. EMBO J 9:833-840) in which the 1.5kbp BamI-XhoI fragment located on exons 2 and 4 was replaced by the XhoI cloning site. Partial sequence of the construct is given in SEQ. ID NO: 4 (Nucleotides 1-2602, MMTHYGP, AC:M12379 (as of 27.4.1993), mouse Thy-1.2 glycoprotein gene (XbaI-BanI),Nucleotides 2603-4982, hβAPP751 SFAD (SmaI/HindIII-Blunted, 2400 bp), AC:X06989 (as of 09.01.2003), (nt:32-2400) with K670N,M671L, Nucleotides 4983-6112, MMTHYGP, AC:M12379 (as of 27.4.1993), XhoI-PolyA site (exon IV)

*PSEN2mut construct:* The FAD mutation N141I was introduced into a human PSEN2 cDNA (SEQ. ID NO: 9, AC: L43964). For expression in transgenic mice, a vector based on the mouse prion gene was used (pPrPHG) (Fischer et al., 1996; Borchelt et al., 1996). A KpnI-NarI fragment, encompassing the prion gene coding region, was deleted and replaced by a unique SceI site. The presenilin 2 coding sequence was inserted by blunt-end ligation and verified by sequencing of the insertion sites.

*Mab11 construct:* A cDNA encoding an immunoglobulin (Ig) heavy chain (H) of the isotype γ1 (SEQ. ID. NO: 10) and a cDNA encoding a light chain (L) of the isotype κ (SEQ. ID. NO: 11) and with specificity for human Ab peptide were used [Bardroff, M.et.al., *Anti-Amyloid beta antibodies and their use.* W003070760]. The cDNAs were amplified in a PCR reaction using the primers in Table 1. The 5' primers contain a SalI (or compatible XhoI) site and the 5' primers contain a BamHI (or compatible BglII) site for directed insertion into the pHSE3' vector [Pircher, H., et al., T cell tolerance to Mlsa encoded antigens in T cell receptor V beta 8.1 chain transgenic mice. Embo J, 1989. 8(3): p. 719-27.]. The PCR-amplified cDNAs were first enzymatically cut with both restriction enzymes SalI and BamHI, and then individually inserted into the corresponding sites of the vector pHSE3'. The expression of the Ig cDNAs in pHSE3' is driven by the murine promoter of the MHC class I gene H-2k and enhanced by the murine Ig H gene enhancer located 3' of the cloned genes [Pircher, H., et al., T cell tolerance to Mlsa encoded antigens in T cell receptor V beta 8.1 chain transgenic mice. Embo J, 1989. 8(3): p. 719-27.]. This expression vector ensures high levels of production of the corresponding inserted gene products in T and B lymphocytes of the transgenic mice ([Pircher, H., et al., T cell tolerance to Mlsa encoded antigens in T cell receptor V beta 8.1 chain transgenic mice. Embo J, 1989. 8(3): p. 719-27.]). The entire expression cassette, including (from 5' to 3'): the H-2k promoter, the inserted cDNA, the poly-A and splice sites and the Ig H gene enhancer element, was then excised from the vector by means of restriction enzyme cut with XhoI and agarose gel purification, and prepared in an adequate concentration for microinjection into fertilized mouse oocytes (2 µg/ml in 10 mM TrisHCl/0.1 mM EDTA, pH 7). The coding potential of the cDNA was confirmed by sequencing the entire cDNAs encoding the anti-Aβ Ig H and L genes.

**Table 1. Sequences of cloning primers.**

| Primer name | Sequence | Restriction site (in bold) | *SEQ. ID No:* |
|---|---|---|---|
| G1.11Salfor (5'IgH) | 5'-ACGT**GTCGA**CGCCGCCACCATGAAACACCTG-3' | SalI (**GTCGAC**) | *12* |
| G1.11 Bamrev (3'IgH) | 5'-ACGT**GGATCC**TCATTTACCCGGAGACAG-3' | BamHI (**GGATCC**) | *13* |
| K.11Xhofor (5'IgL) | 5'-ACGT**CTCGAG**GCCGCCACCATGGTGTTGCAG-3' | XhoI (**CTCGAG**) | *14* |
| K.11Bglrev (3'IgL) | 5'-ACGT**AGATCT**CTAACACTCTCCCCTGTTG-3' | BglII (**AGATCT**) | *15* |

### Example 2. Generation of Transgenic mice

*Generation of B6.PS2APP transgenic mice (line B6.152):* Transgenic animals were generated essentially as described previously (Hogan CF, and Lyacy E. 1995. Manipulating the mouse embryo: a laboratory manual. Ed 2. Plainview, NY: Cold Spring Harbor Laboratory). Vector free linear fragments, carrying Thy-1-huAPP751SFAD or Prp-huPsen2(N141I) constructs were mixed in a 1:1 ratio and microinjected into male pronuclei of C57B1/6 zygotes. After microinjection, viable zygotes were implanted into the oviducts of pseudopregnant B6CBAF1 foster mothers. Integration of the transgenes in the germline was tested in the F1 progeny by detection of the transgenes by PCR. It is generally assumed that co-injected transgenes integrate into the same chromosomal site (Overbeek PA, Aguilar-Cordova E, Hanten G, Schaffner DL, Patel P, Lebowitz RM, and Lieberman MW. 1991. Coinjection strategy for visual identification of transgenic mice. Transgenic Res. 1, 31-37).

*Generation of B6Mab11* (*anti-AβIgG1 transgenic mice):* The transgenic animals were generated as described previously in EP-A-1748294. Fertilized oocytes obtained from C57BL/6 female donors were microinjected with a 1:1 mixture of the purified XhoI fragments encoding for the IgH and L genes described in the previous section (Mab11 construct) to obtain the B6.Mab11 animals. After microinjection, viable zygotes were implanted into the oviducts of pseudopregnant B6CBAF1 foster mothers. Integration of the transgenes in the germline was tested in the F1 progeny by detection of the transgenes by PCR. It is generally assumed that co-injected transgenes integrate into the same chromosomal site (Overbeek PA, Aguilar-Cordova E, Hanten G, Schaffner DL, Patel P, Lebowitz RM, and Lieberman MW. 1991. Coinjection strategy for visual identification of transgenic mice. Transgenic Res. 1, 31-37). In total, 5 double transgenic mice bearing both the transgenic IgH and L genes and 1 single transgenic mouse bearing only the IgH gene were generated and bred.

*Generation of B6.Mab11.PS2APP transgenic mice (Line 86.Mab11.152):* Tansgenic mice having both the transgenes for Mab11 and the human PSEN2mut and APPSw transgenes were generated by crossing males heterozygotes for the Mab11 transgenes with females homozygotes for huPSEN2mut and huAPPSw transgenes. The resulting progeny is either heterozygote for all transgenes or heterozygote for huAPPSw and huPSEN2 only.

### Example 3: PCR analysis

The presence of the transgenes is detected by PCR analysis using genomic DNA isolated from tissue biopsies taken on 10 days old mice. The fragments amplified are specific for the transgenes and do not cross-react with endogenous DNA sequences.

*B6.PS2APP mice (APPswedish-PS2mut double transgenic mice):* The primers 5'-AAG TAT GTC CGC GCA GAA CAG AAG G-3' (SEQ. ID NO: 5) and 5'-CTG AGA TAT TTG AAG GAC TTG GGG AG-3' (SEQ. ID NO: 6) are used to amplify a 932bp fragment overlapping huAPP and muThy1 sequences. Similarly, the primers 5'-TCA TTG GCT TGT GTC TGA CCC T-3' (SEQ. ID NO: 7) and 5'-GCT TTC AAC GTC AGT AGG ACA A-3' (SEQ. ID NO: 8) are used to amplify a 327bp fragment overlapping huPSEN2mut and prion sequences. The huPSEN2 and the huAPPSw transgenes always co-segregate. The PCR reaction was performed using 1µl (about 100 ng) of total DNA obtained from toe clipping tissue, in PCR reaction with the following conditions: 3 min at 95°, 35x [30 sec at 95°, 1 min at 60°, 1 min at 72°], 5 min at 72°. The PCR-amplified DNA fragments of 932 bp for the huAPP trabsgene and of 327bp for the huPS2 transgene were visualized in 1.8 % agarose gels.

*B6.Mab11 mice (Mab11 double transgenic mice):* Pups born from these microinjected embryos were screened for the presence of the transgenes by amplifying genomic DNA prepared from tail biopsies with specific primers. The primers used are indicated in Table 2. The PCR reaction was performed using 1µl (about 100 ng) of total DNA obtained from the tail biopsy, in PCR reaction with the following conditions: 1 min at 90°, 30x [10 sec at 94°, 30 sec at 64°, 90 sec at 72°], 7 min at 72°. The PCR-amplified DNA fragments of about 1270 bp and 660 bp respectively were finally visualized in 1.5 % agarose gels separately for the transgenic IgH and L genes.

**Table 2. Primer sequences for detection of transgenes.**

| **PCR assay** | **Primers** | **PCR fragment** | ***SEQ. ID.*** |
|---|---|---|---|
| Ig H gene | 5H2KP 5'-ATGAATTCACAGTTTCACTTCTGCACC-3' | 1270 bp | *16* |
| | G1.0501 5'-TGTACTCCTTGCCATTCAGC-3' | | *17* |
| Ig L gene | 5H2KP 5'-ATGAATTCACAGTTTCACTTCTGCACC-3 | 660bp | *16* |
| | K.44 5'-GCTCATCAGATGGCGGGAAG-3' | | *18* |

*MAB11.PS2APP mice* (Figure 2): The primers 5'- ATG AAT TCA CAG TTT CAC TTC TGC ACC-3' (SEQ. ID NO: 16) specific to the MHC promoter regions and the primers 5'- TGT ACT CCT TGC CAT TCA GC-3' (SEQ. ID NO: 17) and 5'-GCT CAT CAG ATG GCG GGA AG-3' (SEQ. ID NO: 18) specific to the gamma chain and kappa chain, respectively, are added to the huPSEN2 primers (SEQ. ID NO: 7 and 8) in a multiplex PCR reaction. In the presence of all transgenes, 3 PCR products of different sizes are amplified: 327bp, 600bp and of 1200 bp for the huPSEN2, the hu Ig heavy chain and the hu Ig light chain transgenes, respectively.

The PCR reaction was performed using 1.5µl (about 150 ng) of total DNA obtained from toe clipping tissue, in PCR reaction with the following conditions: 1 min at 95°, 30x [30 sec at 95°, 40 sec at 62°, 80 sec at 72°], 7 min at 72°. PCR-amplified DNA fragments of 327bp, of about 660 bp and 1200 bp were visualized in 1.5 % agarose gels for the huPS2, IgH and L transgenes, respectively.

### Example 4: Immunohistochemistry

Fluothane^{R}-anesthetised 8 months old mice (Wildtype mouse (C57B1/6), Mab11 transgenic mouse, PS2(N141I)/APPsw double transgenic mouse and PS2(N141I)/APPsw/Mab11 quadruple transgenic mouse) were decapitated brains halved and snap frozen in dry ice. Parasagittal cryostat sections between lateral ∼ 1.92 and 1.2 mm according to Paxinos and Franklin were cut at a nominal thickness of 20 micrometer at -18°C using a Leica CM3050 S cryostat and mounted on Superfrost Plus TM slides and stored at -20°C. Staining of amyloid plaques was done by state of the art immunofluorescent staining protocols or staining with Thioflavin-S, a dye specific for amyloid plaques. Briefly, sections were hydrated in PBS and treated with 100% acetone precooled at -20°C for 2 min. Washing with PBS was done twice for two minutes followed by blocking of unspecific binding sites by incubation in 2% bovine serum albumin and 2 % ovalbumin in PBS for 15 min followed by an PBS wash and incubation with a high affinity murine monoclonal antibody (BAP-2), which was conjugated to Alexa488 fluorophore (Molecular Probes)w and incubated at 0.5 mg /ml for 1 hour at room temperature. Staining with Thioflavin-S was done at 0.0125% in ethanol/PBS (40/60 vol %). After rinsing the slides in bidistilled water glass coverslips were mounted using fluorescence mounting medium (S3023, DAKO). Results of 8 month old B6.MAB 11.152 and B6.152 with littermate controls are shown in Figure 8 (BAP-2) and Figure 9 (Thioflavin-S) and demonstrate a complete absence of amyloid deposits in B6.MAB11.152 mice.

The tissue slides from cortex and near hippocampus from B6.MAB 11.152 (20month old) mice and B6.152 mice (20month old) were prepared as described above for the 8 month old animals. The tissue slides were stained as follows:
1. rat/L anti-mouse CD31,10.0µg/ml (Serotec, ID: 1102) in PBS, pH 7.4 + power Block (1x) +10% goat serum (pH7.4), 1h incubation at room temperature, 3x rinsed with DakoCytomation Wash buffer (Tris buffer sodium chloride solution + Tween 20)
2. goat/anti-rat IgG(H+L) ALEXA 488,1:100 (Molecular Probes, ID: 1254) in PBS, pH 7.4 +10% goat serum (pH7.4), 1h incubation at room temperature ; 3x rinsed with DakoCytomation Wash buffer (Tris buffer sodium chloride solution + Tween 20)
3. mouse/BAP-2a ALEXA 555, 5.0µg/ml (EM Labor, ID: 1296) in PBS, pH 7.4 + power block (1x) +10% goat serum (pH7.4), 1h incubation at room temperature ; 3x rinsed with DakoCytomation Wash buffer (Tris buffer sodium chloride solution + Tween 20)

The slides were stained for 5 minutes with DAPI, then rinsed 2x with DakoCytomation Wash buffer, then incubate for 30 minutes with 4 mM CuS04.5H2O in 50 mM CH3COONH4 pH 5.0. After rinsing the slides in bidistilled water glass coverslips were mounted using fluorescence mounting medium (S3023, DAKO).

Results are shown in Figure 14 to 15. In the cortex of B6.MAB 11.152 mice there are occassionally vascular amyloid depositions at thin capillaries. However, of B6.152 mice at the same age showed no no vascular amyloid around cortical capillaries. At larger vessels, like the meningeal and ventricular vessels there was no obvious difference in the degree of vascular amyloidosis observed between B6.MAB11.152 and B6.152 mice.

### Example 5: Western-Blot analysis and Abeta measurements

### Western-Blot analysis

15µg of total protein extract was fractionated by SDS-PAGE and subsequently transferred onto a nitro-cellulose membrane (Amersham, Hybond). For Aβ detection, membranes were heated for 5 min at 95°C in PBS (8.1 mM disodium hydrogen phosphate, 1.5 mM potassium dihydrogen phosphate, 137 mM sodium chloride, 2.7 mM potassium chloride) to increase epitope denaturation and presentation of epitopes, blocked with 5% (w/v) non-fat dry milk in PBST (PBS and 0.05% (v/v) Tween 20), and incubated overnight at 4°C with the primary antibody. For detection of APP, the primary antibody W0-2 (Ida et al., J Biol Chem. 1996, 271(37):22908-1) was used at a concentration of 1 µg/ml. For the detection of presenilin-2 monoclonal antibody PST-20 detecting the amino-terminal fragment was used (Loetscher et al. J Biol Chem. 1997 Aug 15;272(33):20655-9), for tau antibody, Tau-5 (Invitrogen), for total APP antibody Anti-APP-CT20 (Calbiochem) and for human APP the antibody WO-2 which is specific for human APP (Ida et al., J Biol Chem. 1996, 271(37):22908-1). All washing steps were done with PBST at 4°C. Binding of the primary antibody was revealed with horseradish peroxidase conjugated anti-IgG antibody (Amersham) followed by ECL detection system according to the manufacturer's instructions (Amersham).

### Abeta measurements

One brain hemisphere was homogenized in 10 volumes (w/v) of 9M Urea, ,50mM Tris-HCl pH 7.4 containing a cocktail of protease inhibitors (Complete^{™}, Roche Diagnostics GmbH, Mannheim-Germany). Cellular debris were removed by centrifugation at 4°C for 5 min at 1500 g. The protein concentration in the supernatant was measured using the BCA protein Assay (Pierce, USA).

Aβ40 and Aβ42 levels in brain homogenates were determined using liquid phase electrochemiluminescence (LPECL) as described in Czech et al. (J Neurochem, 2007. 101(4): 929-36). Briefly, M-280 paramagnetic beads (Bioveris) were diluted with assay buffer (50 mM Tris, 60 mM NaCl, 0.5% BSA, 1% Tween 20, pH 7.4). 50 µl of brain extract was incubated at room temperature with 50 µl of beads and 25 µl of labeled antibodies (6E10-bio and BAP24-tag for detection of Aβ1-40, 6E10-bio and BAP24-TAG for detection of Aβ1-42) in a final volume of 250 µl for 3 h with gentle shaking. ECL was quantified using an M-Series M8 analyzer (Bioveris). Different dilutions of brain homogenates with assay buffer were measured to assure linear signal response and compared against synthetic standard Aβ1-40 and Aβ1-42 peptides (Bachem). Aβ peptides were solubilized in DMSO at a concentration of 1 mg/ml, aliquoted and stored frozen at -80°C. Results are expressed as ng/mg of brain homogenate referring to the standard curves of Aβ1-40 and Aβ1-42, respectively. Measurements were done in duplicate for each sample dilution and repeated at least two times.

### Example 6: qRT-PCR of mab11 in B6.mab11.152 mice

Spleenocytes were isolated from minced whole spleens by passing through a 70µM cell strainer and serially diluted for RNS extraction. Brain samples were taken with laser microdissections from cortex, cerebellum and subiculum were provided collected into RNAlater®. RNA was extracted using the RNAeasy kit from Qiagen. PCR were performed with the QuantiTect Probe RT-PCR Kit (Qiagen). The TaqMan® Assay (Applied Biosystems Inc) was performed according to the instruction of the manufacturer. The probes were muIGH6_ Mm01718956_ml (IgM = B-cell marker), muAPP Mm00431827_m1 (reference gene for brain), huHC_custom (custom-made by ABI based on mab11 IgG1 const), and huLC_custom (custom-made by ABI based on mab11 IgKappa const).

The PCR was run on the LC480 (Roche Diagnostics). The relative quantitation of the target RNAs were performed by ΔCt method. Brain microdissections from non-trangenic mice gave no positive signal for the Mab11 IgH and IgK RNAs. Results for B6.mab11.152 mice are given in table 3 and 5.

**Table 3: Expression of huIgH and hulgK in brain of B6.mab11.152 mice**

| | **ave** Δ**Cp** | **SD** | ΔΔ | **% of APP** |
|---|---|---|---|---|
| **dlgHCX** | 4.17 | 1.32 | 0.055 | 5.5 |
| **dlgKCx** | 2.41 | 0.58 | 0.188 | 18.8 |
| **dlgHCb** | 4.23 | 0.75 | 0.053 | 5.3 |
| **dlgKCb** | 2.93 | 0.69 | 0.131 | 13.1 |
| **dlgHSub** | 4.32 | 1.12 | 0.050 | 5.0 |
| **dlgKSub** | 2.71 | 1.32 | 0.153 | 15.3 |

The expression for endogenous reference genes IgM and CD20 in wildtype and transgenic mice was cell-number dependent. Only in the transgenic mice robust and cell-number dependent signal for huIg was found. In the wildtype mice no signal was detected vs. assay background (see table 4 and 5).

**Table 4: Ct for B6.mab11.152 mice. IGH6_55, IGH6_56 = endogenous IgM reference, CD20 = endogenous CD20 reference, huIgHG I, huIgKC = Transgene-specific probes.**

| **cell no.** | **IGH6_55** | **IGH6_56** | **CD20** | **huIgHG1** | **hulgKC** |
|---|---|---|---|---|---|
| 10000 | 22.2 | 25.0 | 24.9 | 29.0 | 24.4 |
| 1000 | 25.5 | 28.0 | 28.0 | 32.2 | 27.3 |
| 100 | 28.6 | 31.5 | 30.8 | 35.1 | 30.8 |
| 10 | 31.2 | 34.7 | 32.8 | 37.9 | 34.6 |
| 1 | 32.7 | 38.1 | 33.1 | | 38.0 |

**Table 5: Ct for wildtype C57B1/6. IGH6_55, IGH6_56 = endogenous IgM reference, CD20 = endogenous CD20 reference, huIgHG1, huIgKC = Transgene-specific probes.**

| **cell no.** | **IGH6_55** | **IGH6_56** | **CD20** | **huIgHG1** | **huIgKC** |
|---|---|---|---|---|---|
| 10000 | 20.8 | 23.3 | 23.2 | 44.5 | 38.4 |
| 1000 | 24.0 | 26.4 | 26.4 | | |
| 100 | 27.2 | 29.7 | 29.6 | 38.5 | 36.3 |
| 10 | 30.1 | 33.5 | 32.2 | 38.1 | |
| 1 | 31.9 | 36.5 | 33.5 | | |

## Claims

1. A transgenic mouse whose genome comprises:
a) a first transgenic DNA sequence encoding a human APP Swedish or a human APP London protein, wherein said first transgenic DNA sequence is operably linked to a first promoter;
b) a second transgenic DNA sequence encoding a human Presenilin 2 protein comprising a N141I substitution, wherein said second transgenic DNA sequence is operably linked to a second promoter;
c) a third transgenic DNA sequence encoding a light chain of an antibody directed against the beta-amyloid peptide, wherein said third transgenic DNA sequence is operably linked to a third promoter and;
d) a fourth transgenic DNA sequence encoding a heavy chain of said antibody, wherein said fourth transgenic DNA is operably linked to the third promoter or to a fourth promoter.

2. The transgenic mouse according to claim 1, wherein the third and fourth transgenic DNA sequences encode the light and heavy chain of the anti-Abeta antibody Mab11.

3. The transgenic mouse according to claim 1 or 2, wherein first and second promoters are neuronal promoters.

4. The transgenic mouse according to claim 1 or 2, wherein first and second promoters are brain-specific promoters.

5. The transgenic mouse according to any one of claims 1 to 4, wherein the third and/or fourth promoter is a promoter of the MHC class I gene.

6. The transgenic mouse according to any one of claims 1 to 5, wherein the third and/or fourth promoter is combined with a IgH enhancer

7. The transgenic mouse according to any one of claims 1 to 6, wherein the mouse is homozygous for at least one of the transgenic DNA sequences.

8. Use of the quadruple transgenic mouse according to any one of claims 1 to 7 as model for treatment of Alzheimer's disease.

9. Use of the quadruple transgenic mouse according to any one of claims 1 to 7 as model for preventing Alzheimer's disease.

## Patentansprüche

1. Transgene Maus, deren Genom umfasst:
a) eine erste transgene DNA-Sequenz, die ein humanes APP-Schwedisch- oder ein humanes APP-London-Protein codiert, wobei die erste transgene DNA-Sequenz funktionell mit einem ersten Promotor verknüpft ist;
b) eine zweite transgene DNA-Sequenz, die ein humanes Presenilin-2-Protein umfassend eine N141l-Substitution codiert, wobei die zweite transgene DNA-Sequenz funktionell mit einem zweiten Promotor verknüpft ist;
c) eine dritte transgene DNA-Sequenz, die eine leichte Kette eines Antikörpers codiert, der gegen das beta-Amyloid-Peptid gerichtet ist, wobei die dritte transgene DNA-Sequenz funktionell mit einem dritten Promotor verknüpft ist; und
d) eine vierte transgene DNA-Sequenz, die eine schwere Kette des Antikörpers codiert, wobei die vierte transgene DNA funktionell mit dem dritten oder einem vierten Promotor verknüpft ist.

2. Transgene Maus nach Anspruch 1, wobei die dritte und vierte transgene DNA-Sequenz die leichte und schwere Kette des Anti-Abeta-Antikörpers Mab11 codieren.

3. Transgene Maus nach Anspruch 1 oder 2, wobei erster und zweiter Promotor neuronale Promotoren sind.

4. Transgene Maus nach Anspruch 1 oder 2, wobei erster und zweiter Promotor hirnspezifische Promotoren sind.

5. Transgene Maus nach einem der Ansprüche 1 bis 4, wobei der dritte und/oder vierte Promotor ein Promotor des MHC-Klasse I-Gens ist.

6. Transgene Maus nach einem der Ansprüche 1 bis 5, wobei der dritte und/oder vierte Promotor mit einem IgH-Enhancer kombiniert ist.

7. Transgene Maus nach einem der Ansprüche 1 bis 6, wobei die Maus homozygot für mindestens eine der transgenen DNA-Sequenzen ist.

8. Verwendung der vierfach transgenen Maus nach einem der Ansprüche 1 bis 7 als Modell zur Behandlung der Alzheimer-Krankheit.

9. Verwendung der vierfach transgenen Maus nach einem der Ansprüche 1 bis 7 als Modell zur Prävention der Alzheimer-Krankheit.

## Revendications

1. Souris transgénique le génome comprend :
a) une première séquence d'ADN transgénique codant pour une protéine APP Swedish humaine ou une protéine APP London humaine, la première séquence d'ADN transgénique étant liée de manière fonctionnelle à un premier promoteur ;
b) une deuxième séquence d'ADN transgénique codant pour une protéine Préséniline 2 humaine comprenant une substitution N141I, ladite deuxième séquence d'ADN transgénique étant liée de manière fonctionnelle à un promoteur ;
c) une troisième séquence d'ADN transgénique codant pour une chaîne légère d'un anticorps dirigé contre le peptide bêta-amyloïde, ladite troisième séquence d'ADN transgénique étant liée de manière fonctionnelle à un troisième promoteur ; et
d) une quatrième séquence d'ADN transgénique codant pour une chaîne lourde dudit anticorps, ledit quatrième ADN transgénique étant lié de manière fonctionnelle au troisième promoteur ou à un quatrième promoteur.

2. Souris transgénique suivant la revendication 1, dans laquelle les troisième et quatrième séquences d'ADN transgéniques codent pour la chaîne légère et la chaîne lourde de l'anticorps Mab11 anti-Abéta.

3. Souris transgénique suivant la revendication 1 ou 2, dans laquelle les premier et deuxième promoteurs sont des promoteurs neuronaux.

4. Souris transgénique suivant la revendication 1 ou 2, dans laquelle les premier et deuxième promoteurs sont des promoteurs spécifiques du cerveau.

5. Souris transgénique suivant l'une quelconque des revendications 1 à 4, dans laquelle les troisième et/ou quatrième promoteurs sont un promoteur du gène de MHC de classe 1.

6. Souris transgénique suivant l'une quelconque des revendications 1 à 5, dans laquelle les troisième et/ou quatrième promoteurs sont combinés avec un activateur de IgH.

7. Souris transgénique suivant l'une quelconque des revendications 1 à 6, ladite souris étant homozygote pour au moins une des séquences d'ADN transgénique.

8. Utilisation de la souris transgénique quadruple suivant l'une quelconque des revendications 1 à 7 comme modèle pour le traitement de la maladie d'Alzheimer.

9. Utilisation de la souris transgénique quadruple suivant l'une quelconque des revendications 1 à 7 comme modèle pour la prévention de la maladie d'Alzheimer.


### SEQUENCE LISTING

<110> F. Hoffmann-La Roche AG
<120> quadruple transgenic non-human animal
<130> 24576
<150> EP07119837.8
   <151> 2007-11-01
<160> 19
<170> PatentIn version 3.4
<210> 1
   <211> 2369
   <212> DNA
   <213> Homo sapiens
<300>
   <308> sw/X06989
   <309> 2003-01-09
   <313> (32)..(2369)
<400> 1
   cgggagacgg cggcggtagc ggcgcgggca gagcaaggac gcggcggatc ccactcgcac      60
   agcagcgcac tcggtgcccc gcgcagggtc gcgatgctgc ccggtttggc actgctcctg     120
   ctggccgcct ggacggctcg ggcgctggag gtacccactg atggtaatgc tggcctgctg     180
   gctgaacccc agattgccat gttctgtggc agactgaaca tgcacatgaa tgtccagaat     240
   gggaagtggg attcagatcc atcagggacc aaaacctgca ttgataccaa ggaaggcatc     300
   ctgcagtatt gccaagaagt ctaccctgaa ctgcagatca ccaatgtggt agaagccaac     360
   caaccagtga ccatccagaa ctggtgcaag cggggccgca agcagtgcaa gacccatccc     420
   cactttgtga ttccctaccg ctgcttagtt ggtgagtttg taagtgatgc ccttctcgtt     480
   cctgacaagt gcaaattctt acaccaggag aggatggatg tttgcgaaac tcatcttcac     540
   tggcacaccg tcgccaaaga gacatgcagt gagaagagta ccaacttgca tgactacggc     600
   atgttgctgc cctgcggaat tgacaagttc cgaggggtag agtttgtgtg ttgcccactg     660
   gctgaagaaa gtgacaatgt ggattctgct gatgcggagg aggatgactc ggatgtctgg     720
   tggggcggag cagacacaga ctatgcagat gggagtgaag acaaagtagt agaagtagca     780
   gaggaggaag aagtggctga ggtggaagaa gaagaagccg atgatgacga ggacgatgag     840
   gatggtgatg aggtagagga agaggctgag gaaccctacg aagaagccac agagagaacc     900
   accagcattg ccaccaccac caccaccacc acagagtctg tggaagaggt ggttcgagag     960
   gtgtgctctg aacaagccga gacggggccg tgccgagcaa tgatctcccg ctggtacttt    1020
   gatgtgactg aagggaagtg tgccccattc ttttacggcg gatgtggcgg caaccggaac    1080
   aactttgaca cagaagagta ctgcatggcc gtgtgtggca gcgccattcc tacaacagca    1140
   gccagtaccc ctgatgccgt tgacaagtat ctcgagacac ctggggatga gaatgaacat    1200
   gcccatttcc agaaagccaa agagaggctt gaggccaagc accgagagag aatgtcccag    1260
   gtcatgagag aatgggaaga ggcagaacgt caagcaaaga acttgcctaa agctgataag    1320
   aaggcagtta tccagcattt ccaggagaaa gtggaatctt tggaacagga agcagccaac 1380
   gagagacagc agctggtgga gacacacatg gccagagtgg aagccatgct caatgaccgc 1440
   cgccgcctgg ccctggagaa ctacatcacc gctctgcagg ctgttcctcc tcggcctcgt 1500
   cacgtgttca atatgctaaa gaagtatgtc cgcgcagaac agaaggacag acagcacacc 1560
   ctaaagcatt tcgagcatgt gcgcatggtg gatcccaaga aagccgctca gatccggtcc 1620
   caggttatga cacacctccg tgtgatttat gagcgcatga atcagtctct ctccctgctc 1680
   tacaacgtgc ctgcagtggc cgaggagatt caggatgaag ttgatgagct gcttcagaaa 1740
   gagcaaaact attcagatga cgtcttggcc aacatgatta gtgaaccaag gatcagttac 1800
   ggaaacgatg ctctcatgcc atctttgacc gaaacgaaaa ccaccgtgga gctccttccc 1860
   gtgaatggag agttcagcct ggacgatctc cagccgtggc attcttttgg ggctgactct 1920
   gtgccagcca acacagaaaa cgaagttgag cctgttgatg cccgccctgc tgccgaccga 1980
   ggactgacca ctcgaccagg ttctgggttg acaaatatca agacggagga gatctctgaa 2040
   gtgaagatgg atgcagaatt ccgacatgac tcaggatatg aagttcatca tcaaaaattg 2100
   gtgttctttg cagaagatgt gggttcaaac aaaggtgcaa tcattggact catggtgggc 2160
   ggtgttgtca tagcgacagt gatcgtcatc accttggtga tgctgaagaa gaaacagtac 2220
   acatccattc atcatggtgt ggtggaggtt gacgccgctg tcaccccaga ggagcgccac 2280
   ctgtccaaga tgcagcagaa cggctacgaa aatccaacct acaagttctt tgagcagatg 2340
   cagaactaga cccccgccac agcagcctc 2369
<210> 2
   <211> 5572
   <212> DNA
   <213> Mus musculus
<300>
   <308> SW/X06989
   <309> 2003-01-09
   <313> (1)..(5572)
<400> 2
   tctagagtcc ctccctgctc ccccctctct ccccacccct ggtaaaactg cgggcttcag 60
   gctgggtgca gcaactggag gcgttggcga ccaggaggag gctgcagcta ggggagtcca 120
   ggtgagagca ggccgacggg agggacccgc acatgcaagg accgccgcag ggcgaggatg 180
   caagccttcc ccagctacag ttttgggaaa ggataccagg gcgctcctat atgggggcgc 240
   gggaacctgg ggaaagaagg tgctcccagg tcgaggtggg agaggaaggc agtgcggggt 300
   cacgggcttt ctccctgcta acggacgctt tcgaagagtg ggtgccggag agaaccatga 360
   ggaaggacat caaggacagc ctttggtccc caagctcaaa tcgctttagt ggtgcgaata 420
   gagggaggag gtgggtggca aactggaggg agtccccagc ggtgacctcg tggctggctg 480
   gtgcggggca ccaggtaaga aacgcaatgt tgcgggaggg gactgggtgg caggcgcggg 540
   ggaggggaaa gctagaaagg atgcgaggga gcggaggggg gagggagcgg gggaatctca 600
   actggtagag gaaagttaaa atgaggaaat agcatcaggg tggggttagc caagccgggc 660
   ctcagggaaa gggcgcaaag tttgtctggg tgtgggctta ggtgggctgg gtatgagatt 720
   cggggcgccg aaaacactgc tgcgcctctg ccaaatcacg ctacccctgt atctagttct 780
   gctaggcttc tccagcccca gccccaattc ttttctcagt gtccccttcc ctcccctgaa 840
   tctcaagccc acactccctc ctccataacc cactgttatc aaatctaagt catttgccac 900
   ccaacaacca tcaggaggcg gaagcagacg ggaggagttt gagatcaact tgggctacat 960
   cacgagttcc aagctcacca aggcttctta aggagacctt gtctctaaaa ttaattaatt 1020
   aattaattaa tagtcccctt tctctgccac agaaccttgg gatctggctc ctggtcgcag 1080
   ctccccccac cccaggctga cattcactgc catagcccat ccggaaatcc tagtctattc 1140
   cccatggatc ttgaactgca gagagaatgg cagagtggcc cgccctgtgc aaaggatgtt 1200
   cctagcctag gtggagctcg cgaactcgga gactgtgcct ctcttgggca aggacaggct 1260
   agacagcctg ccggtgttga gctagggcac tgtggggaag gcagagaacc tgtgcagggc 1320
   acgcaatgaa cacaggacca gaaaactgca gccctaggaa cactcaagag ctggccattt 1380
   gcaagcatct ctggcctccg tgcttctcac tcatgtccca tgtcttatac aggcctctgt 1440
   ggcacctcgc ttgcctgatc tcatccctag ccgttaagct ttctgcatga cttatcactt 1500
   ggggcataat gctggatacc taccattttc ttagacccca tcaaaatcct atttgagtgt 1560
   acggttcgga gaactcctta tttatccggt aaatgtcttt tactctgctc tcagggagct 1620
   gaggcaggac attcctgaga tacattggga gaggaataca gtttcaataa aataataggt 1680
   tgggtggagg tacatgccta taatgccacc actcaggaaa tggtggcagc ttcgtgagtt 1740
   tgaggccaac ccaagaaaca tagtgaaacc ctgtcagtaa ataagtaagc aagtatttga 1800
   gtatctacta tatgctaggg ctgacctgga cattaggggt catcttctga acaaactagt 1860
   gcttgaggga ggtatttggg gtttttgttt gtttaatgga tctgaatgag ttccagagac 1920
   tggctacaca gcgatatgac tgagcttaac acccctaaag catacagtca gaccaattag 1980
   acaataaaag gtatgtatag cttaccaaat aaaaaaattg tattttcaag agagtgtctg 2040
   tctgtgtagc cctggctgtt cttgaactca ctctgtagac caggctggcc tggaaatcca 2100
   tctgcctctg cctctctgcc tctctgcctc tctgcctctc tctctgcctc tctctgcctc 2160
   tctctgcccc tctctgcccc tctctgcccc tctctgccgc cctctgcctt ttgccctctg 2220
   ccctctgttc tctggcctct gccctctccc tgccttctcc ctctgcctct gcctcttgag 2280
   tgctggaatc aaaggtgtga gctctatagg tcttaagttc cagaagaaac gtaatgaagt 2340
   cacccagcag gaggtgctca gggacagcaa gacacacaca cccaggacac taggctccca 2400
   cttccttggc tttctctgag tggcaaagga ccttaggcag tgtcactccc taagagaagg 2460
   ggataaagag aggggctgag gtattcagtc atgtgctccg tggatctcaa gccctcaagg 2520
   taaatgggga cccacctgtc ctaccagctg gctgacctgt agctttcccc accacagaat 2580
   ccaagtcgga actcttggca ccatgaaccc agccatcagc gtcgctctcc tgctctcagg 2640
   tactgggcaa gggtcagggc tggcattcta aggaatctgg cttcctccca tcccgggaag 2700
   tagcctcttt gccatagtct caggggcaca ggtggttggg aggtgcgggg gtggggagtg 2760
   gggaggagcc tcaacctcac cagtggtggt ctttgacata ttagaaactc cataatggat 2820
   ctaggaactc ctcttgctgg gtggtggtgg ttgtggtaca cacctttaat ctcagcactc 2880
   aggaggcaga gtcaggtgga tctgttagtc tgaagccagc ctggtctaca gagcaaattc 2940
   caggacagcc agagctattc tcaagataga gaatcccttt cttgaaaaaa ccatttaaaa 3000
   acaaaaacaa aagcaacaca ctcctcttga tctcctgttc ttgaaacaca ttgttgggac 3060
   ccagaacttc agtagattga tggaagttgg agtctgcaag tggtggaaca tcccaccaat 3120
   acctcaaggg cgagtgcaaa ccccacatcc ccccagctca agtctacttt tcctgcaggt 3180
   gggaggcccc gggtctgtgt ctccccaaat tagagaaggc actgctgtgc agtcttgcag 3240
   gtgtcccgag ggcagaaggt gaccagcctg acagcctgcc tggtgaacca aaaccttcgc 3300
   ctggactgcc gccatgagaa taacaccaag gataactcca tccagcatga gttcagcctg 3360
   acccgagaga agaggaagca cgtgctctca ggcacccttg ggattcccga gcacacgtac 3420
   cgctcccgcg tcaccctctc caaccagccc tatatcaagg tccttaccct agccaacttc 3480
   accaccaagg atgagggcga ctacttttgt gagcttcaag tctcgggcgc gaatcccatg 3540
   agctccaata aaagtatcag tgtgtataga ggtgagactg gttcccagaa agataaaatg 3600
   tccaggttag ccaggctggg gtagccaata aaaaaaaaaa aaaaaaacag gcacctccat 3660
   tacccttccc ctaactgctg gtctcctggg aaactgctgc tgtctatgtg agtggggcaa 3720
   gattaggggc cagaaagggg gagcttgtag taaaagcaca gttgaggaaa ctaaatggga 3780
   aaggcagtac agtggtgatt cttgtggtgt ggaggttctg ttacagcatc cggtggagcc 3840
   gctaagatga gaaagcgcca gctagctgcc ttgaaccagc tgacacctgt ctttgcccgc 3900
   ctgagtcctg atctcccctc ctcccggcac cccttctcta tccacagaca agctggtcaa 3960
   gtgtggcggc ataagcctgc tggttcagaa cacatcctgg atgctgctgc tgctgctttc 4020
   cctctccctc ctccaagccc tggacttcat ttctctgtga ctggttgggc ccaaggagaa 4080
   acaggagccc tcgaggtcct tcctctgcag aggtcttgct tctcccggtc agctgactcc 4140
   ctccccaagt ccttcaaata tctcagaaca tggggagaaa cggggacctt gtccctccta 4200
   aggaacccca gtgctgcatg ccatcatccc ccccaccctc gcccccaccc ccgccacttc 4260
   tccctccatg cataccacta gctgtcattt tgtactctgt atttattcta gggctgcttc 4320
   tgattattta gtttgttctt tccctggaga cctgttagaa cataagggcg tatggtgggt 4380
   aggggaggca ggatatcagt cccctggggc gagttcctcc ctgccaacca agccagatgc 4440
   ctgaaagaga tatggatgag ggaagttgga ctgtgcctgt acctggtaca gtcatactct 4500
   gttgaaagaa tcatcgggga gggggggggg ctcaagaggg gagagctctc tgagcctttg 4560
   tggaccatcc aatgaggatg agggcttaga ttctaccagg tcattctcag ccaccacaca 4620
   caagcgctct gccatcactg aagaagcccc ctagggctct tgggccaggg cacactcagt 4680
   aaagatgcag gttcagtcag ggaatgatgg ggaaaggggt aggaggtggg ggagggatca 4740
   ccccctcctc taaaacacga gcctgctgtc tccaaaggcc tctgcctgta gtgagggtgg 4800
   cagaagaaga caaggagcca gaactctgac tccaggatct aagtccgtgc aggaagggga 4860
   tcctagaacc atctggttgg acccagctta ccaagggaga gcctttattc cttctttccc 4920
   ttgcccctct gtgccagccc ctcttgctgt ccctgatccc ccagacagcg agagtcttgc 4980
   aacctgcctc ttccaagacc tcctaatctc aggggcaggc ggtggagtga gatccggcgt 5040
   gcacactttt tggaagatag ctttcccaag gatcctctcc cccactggca gctctgcctg 5100
   tcccatcacc atgtataata ccaccactgc tacagcatct caccgaggaa gaaaatgcac 5160
   aataaaacca agcctctgga gtgtgtcctg gtgtctgtct cttctgtgtc ctggcgtctg 5220
   tctcttctgt gttcttcaag gtcagaaaca aaaaccacac acttcaacct gatggctcgg 5280
   ctgagacttc tgtgtgagaa ggtccaacca gactctgggt accccggccc tccctattcc 5340
   cttgcctcct gtctcccgct tttatagctc cctatgctgg gcttctctgg agagtgaaat 5400
   ctgtgcccga aatcaatcgc attctctctg gtgagtcatc tggcgacagc agttagttca 5460
   cccgccaaca catgggccca gctatgtagc cgagactggc tctggaagtg ccagggactt 5520
   tggtgcataa gtatgtacca tgcccttttt tcacatcgcc ttaactctgc ag 5572
<210> 3
   <211> 4742
   <212> DNA
   <213> Homo sapiens
<400> 3
   gtcgacggat agctttgctc tagactggaa ttcgggcgcg atgctgcccg gtttggcact 60
   gctcctgctg gccgcctgga cggctcgggc gctggaggta cccactgatg gtaatgctgg 120
   cctgctggct gaaccccaga ttgccatgtt ctgtggcaga ctgaacatgc acatgaatgt 180
   ccagaatggg aagtgggatt cagatccatc agggaccaaa acctgcattg ataccaagga 240
   aggcatcctg cagtattgcc aagaagtcta ccctgaactg cagatcacca atgtggtaga 300
   agccaaccaa ccagtgacca tccagaactg gtgcaagcgg ggccgcaagc agtgcaagac 360
   ccatccccac tttgtgattc cctaccgctg cttagttggt gagtttgtaa gtgatgccct 420
   tctcgttcct gacaagtgca aattcttaca ccaggagagg atggatgttt gcgaaactca 480
   tcttcactgg cacaccgtcg ccaaagagac atgcagtgag aagagtacca acttgcatga 540
   ctacggcatg ttgctgccct gcggaattga caagttccga ggggtagagt ttgtgtgttg 600
   cccactggct gaagaaagtg acaatgtgga ttctgctgat gcggaggagg atgactcgga 660
   tgtctggtgg ggcggagcag acacagacta tgcagatgga tccgaagaca aagtagtaga 720
   agtagcagag gaggaagaag tggctgaggt ggaagaagaa gaagccgatg atgacgagga 780
   cgatgaggat ggtgatgagg tagaggaaga ggctgaggaa ccctacgaag aagccacaga 840
   gagaaccacc agcattgcca ccaccaccac caccaccaca gagtctgtgg aagaggtggt 900
   tcgaggtaat ccaccatttg cttggattcc ccccaccccc aaggaaaaga aagcgtaata 960
   ccagagttgg aaatatccac cctagcacca ctgccttccc caatcaaaaa catgtttttt 1020
   tttccaaaag gcttcttatg cttgtgaaat ttttttggtt taacaagcaa acaatttcaa 1080
   ataatgtgaa atctttatta tacagtttgt tttgtacctt gtatatgctg cttggcaaat 1140
   cccaagttaa ttctacaaga ctccgcccaa ccaggtagtc atctaatttg acaaacactg 1200
   agttaggtga ctccttggta ttcctttggc agtgagtgtt tgttatataa tattgtctgt 1260
   taccacctag acgatgctct gagaggtgta aatctctagt cctggtggcc agttaaattc 1320
   ctcagtaaat gtttggtaga tgctgcctaa taaaccagtc caggttgcca ctgggaggat 1380
   taaaagaagt aaacgtgtat acatgaacag agagacagtg ccttttcatg ctaaatgtgg 1440
   ttccccacat ctcctctgat tagaggtgtg ctctgaacaa gccgagacgg ggccgtgccg 1500
   agcaatgatc tcccgctggt actttgatgt gactgaaggg aagtgtgccc cattctttta 1560
   cggcggatgt ggcggcaacc ggaacaactt tgacacagaa gagtactgca tggccgtgtg 1620
   tggcagcgcc agtaagtgga cccttcttcg agcctggcca cctttcgtct ctctcgccac 1680
   tgactctgct ttttgtaaca gattgatttc ctggttcttg ggaatgggcc tgttgctacc 1740
   actaaccaca tttctgtcca cttctctaat tgctcagagt ctccgcagta tgttcaatca 1800
   tgagcacacc tctccgtctt tccctgataa agcatggcca tggatgtgtt ctcttcctag 1860
   ctgtagcaca tatgtcttgc aatccagagg gacttttgag tgcttctctt ttaaacaaag 1920
   ctggagtggc tgttttgtct tctgcaggtc aacttcttac gaaaatagat cttatgttta 1980
   tatgttcatt ttggttttgt tggagggacc aaacctaagt gagtgatttt gtttgttagg 2040
   ttgttttttt gtcagtggac tcgtgcattt cagccatcat tcccatgttt ctctttttgt 2100
   ttttagttat gttctcttat tttttccata gtgtcccaaa gtttactcaa gactacccag 2160
   gaacctcttg cccgagatcc tgttaaacgt acgttgtcat tcacctgagg gaagggaaga 2220
   ggggaggagg atgctgcttg gttcacataa ctccagcatc atcaccttct ttgcatggtt 2280
   ttgtgtttct tgaacacctg tcttagtaaa atgtttcttc ccattacctt gcttgtaatt 2340
   acatctgatt ttgccagaca gcttgagatg ttgggctaag aacatcattg actaagtttc 2400
   ttctatttct gaccaatttc ctttttattt agtctggttt tattgaatat tatgtggaca 2460
   acatcattgt attgtatttg ccattactat tttatttcct aaaagctctc agtgtaactg 2520
   agagcaggct tagcctctca ctgcttttgc agaactgaag aacaagggct aggtgcagtg 2580
   gaaggaaagt gactttactt agcaaagcta gcaatgggga aatggtccag gctcctgcct 2640
   taaagcaacc atctcaaatt ttggattaaa aaacaaaggc ttaaaaaggg gagcttggaa 2700
   tgcagggcat gaaggagggg tgaggaggtg ctgctataca ggacttgttc caaagacttg 2760
   agttattatc cagttcggta agtgggctgg cgcatcccgc acaatcgggt tgtaaattaa 2820
   ctgcagcctt gaggtgatct cctgatgagg gagaattcat tgactattgt caaatttatg 2880
   aagacagaaa agtccttttg tgattcaaaa atgtgtttat tgatgtttat atatcttttg 2940
   tagtgtttcc catactgtct catgaaggtt gtcttcgtaa ttcagttatc taaaatacat 3000
   cctacaattt accttttccc tgggggttgc cgtttattcc tacaacaggc taattacaga 3060
   atatctggtg gtttctgcag gagtcttagt gttatgatat tgatgatatc atccatttac 3120
   tttcaaaatt ggcaaaataa ataattttag gaaagccatg gggatttgga aaaacatgtc 3180
   ttcagcacca actgtttttg ctctttgcat gcttgtttcg taaagaactc taatgctata 3240
   attgcaaaat ggaccatttt aaagattttt ccttcattct gtacttggga gtggtgaaag 3300
   acatccttac tgtgctgcac agtgtctcat ggtgttctct taaacagcat taacgtcttg 3360
   tatgcgctgc tttactaaat tctctgttct gagaaataac tgaaaatacg gctttctatt 3420
   aaacgagtgg attattctgt tgttgttggc ttttttttct caaacctcct tctcttctac 3480
   tttatagttc ctacaacagc agccagtacc cctgatgccg ttgacaagta tctcgagaca 3540
   cctggggatg agaatgaaca tgcccatttc cagaaagcca aagagaggct tgaggccaag 3600
   caccgagaga gaatgtccca ggtcatgaga gaatgggaag aggcagaacg tcaagcaaag 3660
   aacttgccta aagctgataa gaaggcagtt atccagcatt tccaggagaa agtggaatct 3720
   ttggaacagg aagcagccaa cgagagacag cagctggtgg agacacacat ggccagagtg 3780
   gaagccatgc tcaatgaccg ccgccgcctg gccctggaga actacatcac cgctctgcag 3840
   gctgttcctc ctcggcctcg tcacgtgttc aatatgctaa agaagtatgt ccgcgcagaa 3900
   cagaaggaca gacagcacac cctaaagcat ttcgagcatg tgcgcatggt ggatcccaag 3960
   aaagccgctc agatccggtc ccaggttatg acacacctcc gtgtgattta tgagcgcatg 4020
   aatcagtctc tctccctgct ctacaacgtg cctgcagtgg ccgaggagat tcaggatgaa 4080
   gttgatgagc tgcttcagaa agagcaaaac tattcagatg acgtcttggc caacatgatt 4140
   agtgaaccaa ggatcagtta cggaaacgat gctctcatgc catctttgac cgaaacgaaa 4200
   accaccgtgg agctccttcc cgtgaatgga gagttcagcc tggacgatct ccagccgtgg 4260
   cattcttttg gggctgactc tgtgccagcc aacacagaaa acgaagttga gcctgttgat 4320
   gcccgccctg ctgccgaccg aggactgacc actcgaccag gttctgggtt gacaaatatc 4380
   aagacggagg agatctctga agtgaagatg gatgcagaat tccgacatga ctcaggatat 4440
   gaagttcatc atcaaaaatt ggtgttcttt gcagaagatg tgggttcaaa caaaggtgca 4500
   atcattggac tcatggtggg cggtgttgtc atagcgacag tgatcttcat caccttggtg 4560
   atgctgaaga agaaacagta cacatccatt catcatggtg tggtggaggt tgacgccgct 4620
   gtcaccccag aggagcgcca cctgtccaag atgcagcaga acggctacga aaatccaacc 4680
   tacaagttct ttgagcagat gcagaactag acccccgcca cagaagctgg gaattcatcg 4740
   at 4742
<210> 4
   <211> 6112
   <212> DNA
   <213> Artificial
<220>
   <223> description of the artificial sequence: the sequence comprises nucleotides from the mouse Thy-1.2 gylcoprotein gene (XbaI-BanI) and of the human BetaAPP751 comprising the swedish mutation.
<220>
   <221> source
   <222> (1)..(2602)
   <223> mus musculus
<220>
   <221> source
   <222> (2603)..(4982)
   <223> homo sapiens
<220>
   <221> misc_feature
   <222> (2616)..(2616)
   <223> n is a, c, g, or t
<220>
   <221> source
   <222> (4983)..(6112)
   <223> mus musculus
<400> 4
   tctagagtcc ctccctgctc ccccctctct ccccacccct ggtaaaactg cgggcttcag 60
   gctgggtgca gcaactggag gcgttggcga ccaggaggag gctgcagcta ggggagtcca 120
   ggtgagagca ggccgacggg agggacccgc acatgcaagg accgccgcag ggcgaggatg 180
   caagccttcc ccagctacag ttttgggaaa ggataccagg gcgctcctat atgggggcgc 240
   gggaacctgg ggaaagaagg tgctcccagg tcgaggtggg agaggaaggc agtgcggggt 300
   cacgggcttt ctccctgcta acggacgctt tcgaagagtg ggtgccggag agaaccatga 360
   ggaaggacat caaggacagc ctttggtccc caagctcaaa tcgctttagt ggtgcgaata 420
   gagggaggag gtgggtggca aactggaggg agtccccagc ggtgacctcg tggctggctg 480
   gtgcggggca ccaggtaaga aacgcaatgt tgcgggaggg gactgggtgg caggcgcggg 540
   ggaggggaaa gctagaaagg atgcgaggga gcggaggggg gagggagcgg gggaatctca 600
   actggtagag gaaagttaaa atgaggaaat agcatcaggg tggggttagc caagccgggc 660
   ctcagggaaa gggcgcaaag tttgtctggg tgtgggctta ggtgggctgg gtatgagatt 720
   cggggcgccg aaaacactgc tgcgcctctg ccaaatcacg ctacccctgt atctagttct 780
   gctaggcttc tccagcccca gccccaattc ttttctcagt gtccccttcc ctcccctgaa 840
   tctcaagccc acactccctc ctccataacc cactgttatc aaatctaagt catttgccac 900
   ccaacaacca tcaggaggcg gaagcagacg ggaggagttt gagatcaact tgggctacat 960
   cacgagttcc aagctcacca aggcttctta aggagacctt gtctctaaaa ttaattaatt 1020
   aattaattaa tagtcccctt tctctgccac agaaccttgg gatctggctc ctggtcgcag 1080
   ctccccccac cccaggctga cattcactgc catagcccat ccggaaatcc tagtctattc 1140
   cccatggatc ttgaactgca gagagaatgg cagagtggcc cgccctgtgc aaaggatgtt 1200
   cctagcctag gtggagctcg cgaactcgga gactgtgcct ctcttgggca aggacaggct 1260
   agacagcctg ccggtgttga gctagggcac tgtggggaag gcagagaacc tgtgcagggc 1320
   acgcaatgaa cacaggacca gaaaactgca gccctaggaa cactcaagag ctggccattt 1380
   gcaagcatct ctggcctccg tgcttctcac tcatgtccca tgtcttatac aggcctctgt 1440
   ggcacctcgc ttgcctgatc tcatccctag ccgttaagct ttctgcatga cttatcactt 1500
   ggggcataat gctggatacc taccattttc ttagacccca tcaaaatcct atttgagtgt 1560
   acggttcgga gaactcctta tttatccggt aaatgtcttt tactctgctc tcagggagct 1620
   gaggcaggac attcctgaga tacattggga gaggaataca gtttcaataa aataataggt 1680
   tgggtggagg tacatgccta taatgccacc actcaggaaa tggtggcagc ttcgtgagtt 1740
   tgaggccaac ccaagaaaca tagtgaaacc ctgtcagtaa ataagtaagc aagtatttga 1800
   gtatctacta tatgctaggg ctgacctgga cattaggggt catcttctga acaaactagt 1860
   gcttgaggga ggtatttggg gtttttgttt gtttaatgga tctgaatgag ttccagagac 1920
   tggctacaca gcgatatgac tgagcttaac acccctaaag catacagtca gaccaattag 1980
   acaataaaag gtatgtatag cttaccaaat aaaaaaattg tattttcaag agagtgtctg 2040
   tctgtgtagc cctggctgtt cttgaactca ctctgtagac caggctggcc tggaaatcca 2100
   tctgcctctg cctctctgcc tctctgcctc tctgcctctc tctctgcctc tctctgcctc 2160
   tctctgcccc tctctgcccc tctctgcccc tctctgccgc cctctgcctt ttgccctctg 2220
   ccctctgttc tctggcctct gccctctccc tgccttctcc ctctgcctct gcctcttgag 2280
   tgctggaatc aaaggtgtga gctctatagg tcttaagttc cagaagaaac gtaatgaagt 2340
   cacccagcag gaggtgctca gggacagcaa gacacacaca cccaggacac taggctccca 2400
   cttccttggc tttctctgag tggcaaagga ccttaggcag tgtcactccc taagagaagg 2460
   ggataaagag aggggctgag gtattcagtc atgtgctccg tggatctcaa gccctcaagg 2520
   taaatgggga cccacctgtc ctaccagctg gctgacctgt agctttcccc accacagaat   2580
   ccaagtcgga actcttggca cctagaggat ctcganggga gacggcggcg gtggcggcgc   2640
   gggcagagca aggacgcggc ggatcccact cgcacagcag cgcactcggt gccccgcgca 2700
   gggtcgcgat gctgcccggt ttggcactgc tcctgctggc cgcctggacg gctcgggcgc 2760
   tggaggtacc cactgatggt aatgctggcc tgctggctga accccagatt gccatgttct 2820
   gtggcagact gaacatgcac atgaatgtcc agaatgggaa gtgggattca gatccatcag 2880
   ggaccaaaac ctgcattgat accaaggaag gcatcctgca gtattgccaa gaagtctacc 2940
   ctgaactgca gatcaccaat gtggtagaag ccaaccaacc agtgaccatc cagaactggt 3000
   gcaagcgggg ccgcaagcag tgcaagaccc atccccactt tgtgattccc taccgctgct 3060
   tagttggtga gtttgtaagt gatgcccttc tcgttcctga caagtgcaaa ttcttacacc 3120
   aggagaggat ggatgtttgc gaaactcatc ttcactggca caccgtcgcc aaagagacat 3180
   gcagtgagaa gagtaccaac ttgcatgact acggcatgtt gctgccctgc ggaattgaca 3240
   agttccgagg ggtagagttt gtgtgttgcc cactggctga agaaagtgac aatgtggatt 3300
   ctgctgatgc ggaggaggat gactcggatg tctggtgggg cggagcagac acagactatg 3360
   cagatgggag tgaagacaaa gtagtagaag tagcagagga ggaagaagtg gctgaggtgg 3420
   aagaagaaga agccgatgat gacgaggacg atgaggatgg tgatgaggta gaggaagagg 3480
   ctgaggaacc ctacgaagaa gccacagaga gaaccaccag cattgccacc accaccacca 3540
   ccaccacaga gtctgtggaa gaggtggttc gagaggtgtg ctctgaacaa gccgagacgg 3600
   ggccgtgccg agcaatgatc tcccgctggt actttgatgt gactgaaggg aagtgtgccc 3660
   cattctttta cggcggatgt ggcggcaacc ggaacaactt tgacacagaa gagtactgca 3720
   tggccgtgtg tggcagcgcc attcctacaa cagcagccag tacccctgat gccgttgaca 3780
   agtatctcga gacacctggg gatgagaatg aacatgccca tttccagaaa gccaaagaga 3840
   ggcttgaggc caagcaccga gagagaatgt cccaggtcat gagagaatgg gaagaggcag 3900
   aacgtcaagc aaagaacttg cctaaagctg ataagaaggc agttatccag catttccagg 3960
   agaaagtgga atctttggaa caggaagcag ccaacgagag acagcagctg gtggagacac 4020
   acatggccag agtggaagcc atgctcaatg accgccgccg cctggccctg gagaactaca 4080
   tcaccgctct gcaggctgtt cctcctcggc ctcgtcacgt gttcaatatg ctaaagaagt 4140
   atgtccgcgc agaacagaag gacagacagc acaccctaaa gcatttcgag catgtgcgca 4200
   tggtggatcc caagaaagcc gctcagatcc ggtcccaggt tatgacacac ctccgtgtga 4260
   tttatgagcg catgaatcag tctctctccc tgctctacaa cgtgcctgca gtggccgagg 4320
   agattcagga tgaagttgat gagctgcttc agaaagagca aaactattca gatgacgtct 4380
   tggccaacat gattagtgaa ccaaggatca gttacggaaa cgatgctctc atgccatctt 4440
   tgaccgaaac gaaaaccacc gtggagctcc ttcccgtgaa tggagagttc agcctggacg 4500
   atctccagcc gtggcattct tttggggctg actctgtgcc agccaacaca gaaaacgaag 4560
   ttgagcctgt tgatgcccgc cctgctgccg accgaggact gaccactcga ccaggttctg 4620
   ggttgacaaa tatcaagacg gaggagatct ctgaagtgaa tctggatgca gaattccgac 4680
   atgactcagg atatgaagtt catcatcaaa aattggtgtt ctttgcagaa gatgtgggtt 4740
   caaacaaagg tgcaatcatt ggactcatgg tgggcggtgt tgtcatagcg acagtgatcg 4800
   tcatcacctt ggtgatgctg aagaagaaac agtacacatc cattcatcat ggtgtggtgg 4860
   aggttgacgc cgctgtcacc ccagaggagc gccacctgtc caagatgcag cagaacggct 4920
   acgaaaatcc aacctacaag ttctttgagc agatgcagaa ctagaccccc gccacagaag 4980
   cttcgaggtc cttcctctgc agaggtcttg cttctcccgg tcagctgact ccctccccaa 5040
   gtccttcaaa tatctcagaa catggggaga aacggggacc ttgtccctcc taaggaaccc 5100
   cagtgctgca tgccatcatc ccccccaccc tcgcccccac ccccgccact tctccctcca 5160
   tgcataccac tagctgtcat tttgtactct gtatttattc tagggctgct tctgattatt 5220
   tagtttgttc tttccctgga gacctgttag aacataaggg cgtatggtgg gtaggggagg 5280
   caggatatca gtcccctggg gcgagttcct ccctgccaac caagccagat gcctgaaaga 5340
   gatatggatg agggaagttg gactgtgcct gtacctggta cagtcatact ctgttgaaag 5400
   aatcatcggg gagggggggg ggctcaagag gggagagctc tctgagcctt tgtggaccat 5460
   ccaatgagga tgagggctta gattctacca ggtcattctc agccaccaca cacaagcgct 5520
   ctgccatcac tgaagaagcc ccctagggct cttgggccag ggcacactca gtaaagatgc 5580
   aggttcagtc agggaatgat ggggaaaggg gtaggaggtg ggggagggat caccccctcc 5640
   tctaaaacac gagcctgctg tctccaaagg cctctgcctg tagtgagggt ggcagaagaa 5700
   gacaaggagc cagaactctg actccaggat ctaagtccgt gcaggaaggg gatcctagaa 5760
   ccatctggtt ggacccagct taccaaggga gagcctttat tccttctttc ccttgcccct 5820
   ctgtgccagc ccctcttgct gtccctgatc ccccagacag cgagagtctt gcaacctgcc 5880
   tcttccaaga cctcctaatc tcaggggcag gcggtggagt gagatccggc gtgcacactt 5940
   tttggaagat agctttccca aggatcctct cccccactgg cagctctgcc tgtcccatca 6000
   ccatgtataa taccaccact gctacagcat ctcaccgagg aagaaaatgc acaataaaac 6060
   caagcctctg gagtgtgtcc tggtgtctgt ctcttctgtg tcctggcgtc tg 6112
<210> 5
   <211> 25
   <212> DNA
   <213> Artificial
<220>
   <223> Description of artificial sequence: PCR primer
<400> 5
   aagtatgtcc gcgcagaaca gaagg 25
<210> 6
   <211> 26
   <212> DNA
   <213> Artificial
<220>
   <223> Description of artificial sequence: PCR primer
<400> 6
   ctgagatatt tgaaggactt ggggag 26
<210> 7
   <211> 22
   <212> DNA
   <213> Artificial
<220>
   <223> Description of artificial description: PCR primer
<400> 7
   tcattggctt gtgtctgacc ct 22
<210> 8
   <211> 22
   <212> DNA
   <213> Artificial
<220>
   <223> Description of artificial sequence: PCR primer
<400> 8
   gctttcaacg tcagtaggac aa 22
<210> 9
   <211> 2236
   <212> DNA
   <213> Homo sapiens
<300>
   <308> X06989
   <309> 2003-01-09
   <313> (1)..(2236)
<400> 9
   cgagcggcgg cggagcaggc atttccagca gtgaggagac agccagaagc aagctattgg 60
   agctgaagga acctgagaca gaagctagtc ccccctctga attttactga tgaagaaact 120
   gaggccacag agctaaagtg acttttccca aggtcgccca gcgaggacgt gggacttctc 180
   agacgtcagg agagtgatgt gagggagctg tgtgaccata gaaagtgacg tgttaaaaac 240
   cagcgctgcc ctctttgaaa gccagggagc atcattcatt tagcctgctg agaagaagaa 300
   accaagtgtc cgggattcag acctctctgc ggccccaagt gttcgtggtg cttccagagg 360
   cagggctatg ctcacattca tggcctctga cagcgaggaa gaagtgtgtg atgagcggac 420
   gtccctaatg tcggccgaga gccccacgcc gcgctcctgc caggagggca ggcagggccc 480
   agaggatgga gagaacactg cccagtggag aagccaggag aacgaggagg acggtgagga 540
   ggaccctgac cgctatgtct gtagtggggt tcccgggcgg ccgccaggcc tggaggaaga 600
   gctgaccctc aaatacggag cgaagcacgt gatcatgctg tttgtgcctg tcactctgtg 660
   catgatcgtg gtggtagcca ccatcaagtc tgtgcgcttc tacacagaga agaatggaca 720
   gctcatctac acgacattca ctgaggacac accctcggtg ggccagcgcc tcctcaactc 780
   cgtgctgaac accctcatca tgatcagcgt catcgtggtt atgaccatct tcttggtggt 840
   gctctacaag taccgctgct acaagttcat ccatggctgg ttgatcatgt cttcactgat 900
   gctgctgttc ctcttcacct atatctacct tggggaagtg ctcaagacct acaatgtggc 960
   catggactac cccaccctct tgctgactgt ctggaacttc ggggcagtgg gcatggtgtg 1020
   catccactgg aagggccctc tggtgctgca gcaggcctac ctcatcatga tcagtgcgct 1080
   catggcccta gtgttcatca agtacctccc agagtggtcc gcgtgggtca tcctgggcgc 1140
   catctctgtg tatgatctcg tggctgtgct gtgtcccaaa gggcctctga gaatgctggt 1200
   agaaactgcc caggagagaa atgagcccat attccctgcc ctgatatact catctgccat 1260
   ggtgtggacg gttggcatgg cgaagctgga cccctcctct cagggtgccc tccagctccc 1320
   ctacgacccg gagatggaag aagactccta tgacagtttt ggggagcctt cataccccga 1380
   agtctttgag cctcccttga ctggctaccc aggggaggag ctggaggaag aggaggaaag 1440
   gggcgtgaag cttggcctcg gggacttcat cttctacagt gtgctggtgg gcaaggcggc 1500
   tgccacgggc agcggggact ggaataccac gctggcctgc ttcgtggcca tcctcattgg 1560
   cttgtgtctg accctcctgc tgcttgctgt gttcaagaag gcgctgcccg ccctccccat 1620
   ctccatcacg ttcgggctca tcttttactt ctccacggac aacctggtgc ggccgttcat 1680
   ggacaccctg gcctcccatc agctctacat ctgagggaca tggtgtgcca caggctgcaa 1740
   gctgcaggga attttcattg gatgcagttg tatagtttta cactctagtg ccatatattt 1800
   ttaagacttt tctttcctta aaaaataaag tacgtgttta cttggtgagg aggaggcaga 1860
   accagctctt tggtgccagc tgtttcatca ccagactttg gctcccgctt tggggagcgc 1920
   ctcgcttcac ggacaggaag cacagcaggt ttatccagat gaactgagaa ggtcagatta 1980
   gggcggggag aagagcatcc ggcatgaggg ctgagatgcg caaagagtgt gctcgggagt 2040
   ggcccctggc acctgggtgc tctggctgga gaggaaaagc cagttcccta cgaggagtgt 2100
   tcccaatgct ttgtccatga tgtccttgtt attttattgc ctttagaaac tgagtcctgt 2160
   tcttgttacg gcagtcacac tgctgggaag tggcttaata gtaatatcaa taaatagatg 2220
   agtcctgtta gaaaaa 2236
<210> 10
   <211> 1562
   <212> DNA
   <213> Homo sapiens
<400> 10
   gcgccaccat gaaacacctg tggttcttcc tcctgctggt ggcagctccc agatgggtcc 60
   tgtcccaggt ggaattggtg gaaagcggcg gcggcctggt gcaaccgggc ggcagcctgc 120
   gtctgagctg cgcggcctcc ggatttacct ttagcagcta tgcgatgagc tgggtgcgcc 180
   aagcccctgg gaagggtcta gagtgggtga gcggtattaa tgctgctggt tttcgtactt 240
   attatgctga ttctgttaag ggtcgtttta ccatttcacg tgataattcg aaaaacaccc 300
   tgtatctgca aatgaacagc ctgcgtgcgg aagatacggc cgtgtattat tgcgcgcgtg 360
   gtaagggtaa tactcataag ccttatggtt atgttcgtta ttttgatgtt tggggccaag 420
   gcaccctggt gacggttagc tcagcctcca ccaagggccc atcggtcttc cccctggcac 480
   cctcctccaa gagcacctct gggggcacag cagccctggg ctgcctggtc aaggactact 540
   tccccgaacc ggtgacggtg tcgtggaact caggcgccct gaccagcggc gtgcacacct 600
   tcccggctgt cctacagtcc tcaggactct actccctcag cagcgtggtg accgtgccct 660
   ccagcagctt gggcacccag acctacatct gcaacgtgaa tcacaagccc agcaacacca 720
   aggtggacaa gaaagttgag cccaaatctt gtgacaaaac tcacacatgc ccaccgtgcc 780
   cagcacctga actcctgggg ggaccgtcag tcttcctctt ccccccaaaa cccaaggaca 840
   ccctcatgat ctcccggacc cctgaggtca catgcgtggt ggtggacgtg agccacgaag 900
   accctgaggt caagttcaac tggtacgtgg acggcgtgga ggtgcataat gccaagacaa 960
   agccgcggga ggagcagtac aacagcacgt accgtgtggt cagcgtcctc accgtcctgc 1020
   accaggactg gctgaatggc aaggagtaca agtgcaaggt ctccaacaaa gccctcccag 1080
   cccccatcga gaaaaccatc tccaaagcca aagggcagcc ccgagaacca caggtgtaca 1140
   ccctgccccc atcccgggat gagctgacca agaaccaggt cagcctgacc tgcctggtca 1200
   aaggcttcta tcccagcgac atcgccgtgg agtgggagag caatgggcag ccggagaaca 1260
   actacaagac cacgcctccc gtgctggact ccgacggctc cttcttcctc tacagcaagc 1320
   tcaccgtgga caagagcagg tggcagcagg ggaacgtctt ctcatgctcc gtgatgcatg 1380
   aggctctgca caaccactac acacagaaga gcctctccct gtctccgggt aaatgagtgc 1440
   cacggccggc aagcccccgc tccccaggct ctcggggtcg cgcgaggatg cttggcacgt 1500
   accccgtgta catacttccc aggcacccag catggaaata aagcacccag cgcttccctg 1560
   gg 1562
<210> 11
   <211> 715
   <212> DNA
   <213> Homo sapiens
<400> 11
   cgccaccatg gtgttgcaga cccaggtctt catttctctg ttgctctgga tctctggtgc 60
   ctacggggat atcgtgctga cccagagccc ggcgaccctg agcctgtctc cgggcgaacg 120
   tgcgaccctg agctgcagag cgagccagta tgttgatcgt acttatctgg cgtggtacca 180
   gcagaaacca ggtcaagcac cgcgtctatt aatttatggc gcgagcagcc gtgcaactgg 240
   ggtcccggcg cgttttagcg gctctggatc cggcacggat tttaccctga ccattagcag 300
   cctggaacct gaagactttg cgacttatta ttgccagcag atttattctt ttcctcatac 360
   ctttggccag ggtacgaaag ttgaaattaa acgtacggtg gctgcaccat ctgtcttcat 420
   cttcccgcca tctgatgagc agttgaaatc tggaactgcc tctgttgtgt gcctgctgaa 480
   taacttctat cccagagagg ccaaagtaca gtggaaggtg gataacgccc tccaatcggg 540
   taactcccag gagagtgtca cagagcagga cagcaaggac agcacctaca gcctcagcag 600
   caccctgacg ctgagcaaag cagactacga gaaacacaaa gtctacgcct gcgaagtcac 660
   ccatcagggc ctgagctcgc ccgtcacaaa gagcttcaac aggggagagt gttag 715
<210> 12
   <211> 31
   <212> DNA
   <213> Artificial
<220>
   <223> Description of artificial sequence: PCR primer
<400> 12
   acgtgtcgac gccgccacca tgaaacacct g 31
<210> 13
   <211> 28
   <212> DNA
   <213> Artificial
<220>
   <223> description of artificial sequence: PCR primer
<400> 13
   acgtggatcc tcatttaccc ggagacag 28
<210> 14
   <211> 31
   <212> DNA
   <213> Artificial
<220>
   <223> Description of artificial sequence: PCR primer
<400> 14
   acgtctcgag gccgccacca tggtgttgca g 31
<210> 15
   <211> 29
   <212> DNA
   <213> Artificial
<220>
   <223> Description of artificial sequence: PCR primer
<400> 15
   acgtagatct ctaacactct cccctgttg 29
<210> 16
   <211> 27
   <212> DNA
   <213> Artificial
<220>
   <223> Description of artificial sequence: PCR primer
<400> 16
   atgaattcac agtttcactt ctgcacc 27
<210> 17
   <211> 20
   <212> DNA
   <213> Artificial
<220>
   <223> Description of artificial sequence: PCR primer
<400> 17
   tgtactcctt gccattcagc 20
<210> 18
   <211> 20
   <212> DNA
   <213> Artificial
<220>
   <223> Description of artificial sequence: PCR primer
<400> 18
   gctcatcaga tggcgggaag 20
<210> 19
   <211> 448
   <212> PRT
   <213> Homo sapiens
<400> 19
   Met Leu Thr Phe Met Ala Ser Asp Ser Glu Glu Glu Val Cys Asp Glu
   1               5                   10                  15
   Arg Thr Ser Leu Met Ser Ala Glu Ser Pro Thr Pro Arg Ser Cys Gln
               20                  25                  30
   Glu Gly Arg Gln Gly Pro Glu Asp Gly Glu Asn Thr Ala Gln Trp Arg
           35                  40                  45
   Ser Gln Glu Asn Glu Glu Asp Gly Glu Glu Asp Pro Asp Arg Tyr Val
       50                  55                  60
   Cys Ser Gly Val Pro Gly Arg Pro Pro Gly Leu Glu Glu Glu Leu Thr
   65                  70                  75                  80
   Leu Lys Tyr Gly Ala Lys His Val Ile Met Leu Phe Val Pro Val Thr
                   85                  90                  95
   Leu Cys Met Ile Val Val Val Ala Thr Ile Lys Ser Val Arg Phe Tyr
               100                 105                 110
   Thr Glu Lys Asn Gly Gln Leu Ile Tyr Thr Thr Phe Thr Glu Asp Thr
           115                 120                 125
   Pro Ser Val Gly Gln Arg Leu Leu Asn Ser Val Leu Ile Thr Leu Ile
       130                 135                 140
   Met Ile Ser Val Ile Val Val Met Thr Ile Phe Leu Val Val Leu Tyr
   145                 150                 155                 160
   Lys Tyr Arg Cys Tyr Lys Phe Ile His Gly Trp Leu Ile Met Ser Ser
                   165                 170                 175
   Leu Met Leu Leu Phe Leu Phe Thr Tyr Ile Tyr Leu Gly Glu Val Leu
               180                 185                 190
   Lys Thr Tyr Asn Val Ala Met Asp Tyr Pro Thr Leu Leu Leu Thr Val
           195                 200                 205
   Trp Asn Phe Gly Ala Val Gly Met Val Cys Ile His Trp Lys Gly Pro
       210                 215                 220
   Leu Val Leu Gln Gln Ala Tyr Leu Ile Met Ile Ser Ala Leu Met Ala
   225                 230                 235                 240
   Leu Val Phe Ile Lys Tyr Leu Pro Glu Trp Ser Ala Trp Val Ile Leu
                   245                 250                 255
   Gly Ala Ile Ser Val Tyr Asp Leu Val Ala Val Leu Cys Pro Lys Gly
               260                 265                 270
   Pro Leu Arg Met Leu Val Glu Thr Ala Gln Glu Arg Asn Glu Pro Ile
           275                 280                 285
   Phe Pro Ala Leu Ile Tyr Ser Ser Ala Met Val Trp Thr Val Gly Met
       290                 295                 300
   Ala Lys Leu Asp Pro Ser Ser Gln Gly Ala Leu Gln Leu Pro Tyr Asp
   305                 310                 315                 320
   Pro Glu Met Glu Glu Asp Ser Tyr Asp Ser Phe Gly Glu Pro Ser Tyr
                   325                 330                 335
   Pro Glu Val Phe Glu Pro Pro Leu Thr Gly Tyr Pro Gly Glu Glu Leu
               340                 345                 350
   Glu Glu Glu Glu Glu Arg Gly Val Lys Leu Gly Leu Gly Asp Phe Ile
           355                 360                 365
   Phe Tyr Ser Val Leu Val Gly Lys Ala Ala Ala Thr Gly Ser Gly Asp
       370                 375                 380
   Trp Asn Thr Thr Leu Ala Cys Phe Val Ala Ile Leu Ile Gly Leu Cys
   385                 390                 395                 400
   Leu Thr Leu Leu Leu Leu Ala Val Phe Lys Lys Ala Leu Pro Ala Leu
                   405                 410                 415
   Pro Ile Ser Ile Thr Phe Gly Leu Ile Phe Tyr Phe Ser Thr Asp Asn
               420                 425                 430
   Leu Val Arg Pro Phe Met Asp Thr Leu Ala Ser His Gln Leu Tyr Ile
           435                 440                 445